# EUROPEAN PATENT APPLICATION

(11) **EP 1 285 970 A2**
(43) Date of publication of application: **26.02.2003**
(21) Application number: 02013926.7
(22) Date of filing: 24.06.2002
(51) Int. Cl.: C12Q 1/68

(54) **Metastasis-associated genes**

(30) Priority: 26.06.2001 US 300991 P
(71) Applicant: National Taiwan University, Taipei (TW); Academia Sinica, Nan-Kang, Taipei (TW); National Health Research Institutes, Taipei, 11529 (TW)
(72) Inventor: Chen, Jeremy J.W., Feng-Yuan, Taichung Hsien (TW); Yang, Pan-Chyr, Hsin-Sheng S. Rd., Taipei (TW); Peck, Konan, Fu-Hsing S. Rd., Taipei (TW); Hong, Tse-Ming, Taipei (TW); Yang, Shuenn-Chen, 3rd Fl., No. 9-1, Alley 2, Taipei (TW); Wu, Cheng-Wen, Nei-Hu, Taipei (TW)
(74) Representative: Reinhard - Skuhra - Weise & Partner

(57) **Abstract**

Many genes are identified as being metastasis associated. Identifying and profiling of these genes expression can be used to evaluate a sample, to diagnose tumor invasive potential or metastatic development in a sample, or screen for a test compound useful in the prevention or treatment of tumor metastasis.

## Description

### CROSS-REFERENCE TO RELATED APPLICATIONS

This application claims priority to U.S. Application Serial No. 60/300,991, filed June 26, 2002, the contents of which is hereby incorporated by reference in its entirety for all purposes.

### BACKGROUND

This application relates to metastasis-associated genes. Metastasis is a multiple-step process that includes the migration of cancer cells from a primary tumor site to a remote site. This process involves interactions between cancer cells and their surrounding microenvironment. Metastasis is a major cause of mortality for cancer patients. Many studies on cancer metastasis have been conducted and several molecules participating in tumor cell invasion and metastasis have been identified and characterized. Among these molecules, some facilitate invasion and metastasis, e.g. laminin receptor, vitronectin receptor, metalloproteinases, and CD44; while others inhibit these processes, e.g. cadherin, tissue inhibitors of metalloproteinases, and nm23. See, e.g., Wewer *et al.* (1986) *Proc. Natl. Acad. Sci. USA* 83: 7137-7141; Albelda *et al.* (1990) *Cancer Res.* 50: 6757-6764; Powell *et al.* (1993) *Cancer Res.* 53: 417-422; Sreenath *et al*. (1992) *Cancer Res.* 52: 4942-4947; Birch *et al.* (1991) *Cancer Res.* 51: 6660-6667; Harn *et al*. (1996) *J. Clin. Gastroenterol*. *22:* 107-110; Uleminckx *et al.* (1991) *Cell* 66: 107-119; Liotta et *al.* (1991) *Cell* 64: 327-336; Goldberg *et al*. (1989) *Proc. Natl. Acad. Sci. USA* 86: 8207-8211; and Kodera *et al.* (1994) *Cancer* 73: 259-265.

Calcyclin and AXL are also associated with metastasis (Weterman *et al*. (1992) *Cancer Res.* 52: 1291-1296; and Jacob *et al*. (1999) *Cancer Detect. Prev.* 23: 325-332). Some proteases and adhesion molecules including disintegrin-metalloprotease, MMP-19 protein, interstitial collagenase, protocadherin, integrin α-3 and integrin α-6 have been reported before to be associated with cancer invasion and metastasis. See, for example, Kanamori *et al*. (1999) *Cancer Res.* 59: 4225-4227; Okada *et al*. (1994) *Clin. Exp. Metastas.* 12: 305-314; Morini *et al*. (2000) *Int. J. Cancer*, 87: 336-342; and Friedrichs *et al.* (1995) *Cancer Res.* 55: 901-906. The tumor-associated antigen L6 is reportedly highly expressed on several carcinomas such as lung and breast cancer (Marken *et al*. (1992) *Proc. Natl. Acad. Sci. USA* 89: 3503-3507).

### SUMMARY

This invention is based on the discovery that the genes listed in the four groups -- Group I, II, III, and IV -- are associated with tumor metastasis.

As used herein, "Group I" refers to the group consisting the genes listed in Table I. "Group II" refers to the group consisting of the genes listed in Table II. "Group III" refers to the group consisting of the genes listed in Table III. "Group IV" refers to the group consisting of the genes listed in Table IV.

Each of the genes in these groups are either negatively or positively correlated with invasiveness. Group I, II, III, or IV genes (all or a fraction of Group I, II, III, or IV) can be used independently, or in combination. Each of the terms "Group I," "Group II," and "Group III" encompasses polypeptides encoded by the listed genes. Whether the terms refer to genes, polypeptides, or both will be apparent from context.

In one aspect, this invention features a first method of evaluating a sample. The method includes determining the level of the expression of at least one nucleic acid selected from Group I, II, III, or IV in a sample, and comparing the level of expression to a reference expression value to thereby evaluate the sample. The level of expression can be a value that is compared to a reference value to thereby evaluate the sample. The reference value can be arbitrary or associated with a reference sample or a reference state.

The sample used to obtain the reference expression can be one or more of: (1) a sample from a normal subject; (2) a sample suspected of having or having a disorder, e.g., a neoplastic disorder, or metastatic disorder; (3) a sample from a subject having a metastatic disorder and undergoing treatment; and (4) a sample from a subject being evaluated, e.g., an earlier sample or a normal sample of the same subject. An expression, e.g., a sample expression or a reference expression, can be a qualitative or quantitative assessment of the abundance of (1) an mRNA transcribed from a nucleic acid; or of (2) a polypeptide encoded by the nucleic acid. The mRNA expression can be determined by, for example, quantitative PCR, Northern analysis, microarray analysis, serial analysis of nucleic acid expression (SAGE), and other routine methods. Polypeptide expression can be determined by antibody probes, e.g., using an antibody array, or by quantitative mass spectroscopy.

In some embodiments, the method can further include determining the levels of expression of 10%, 20%, 30%, 50%, 75%, 80%, 90%, 99% or more nucleic acids selected from Groups I, II or III.

In another aspect, the invention features a second method of evaluating a sample. The method includes identifying a sample expression profile, wherein the sample expression profile represents the levels of expression of at least two nucleic acids selected from Group I, II, III, and/or IV; and comparing the sample expression profile to at least one reference expression profile to thereby evaluate the sample.

An "expression profile" (e.g., a reference expression profile or a sample expression profile) used herein includes a plurality of values, wherein each value corresponds to the level of expression of a different nucleic acid, splice-variant or allelic variant of a nucleic acid or a translation product thereof. The value can be a qualitative or quantitative assessment of the abundance of (1) an mRNA transcribed from a nucleic acid; or of (2) a polypeptide encoded by the nucleic acid. An expression profile has a plurality of values, at least two of which correspond to a nucleic acid of Group I, II, III, or IV.

In some embodiments, the expression profile can include values for 50%, 60%, 80%, or 90% of the nucleic acids selected from Groups I and II. Alternatively, the expression profile can include values for all the nucleic acids selected from Group II, or a fraction of the nucleic acids of Group II, e.g., 20%, 40%, 50%, 60%, 80%, or 90% of nucleic acids of Group II.

In some other embodiments, the expression profile can include values for all the nucleic acids of Group I, II, III, and/or IV (i.e., 100% of the nucleic acids), or a fraction of the nucleic acids of Group I, II, III, and/or IV, e.g., at least 20%, 40%, 50%, 60%, 80%, or 90% of the nucleic acids of Group I, II, III, and/or IV. The expression profile can be obtained from an array by a method, which may include providing an array; contacting the array with a nucleic acid mixture (e.g., a mixture of nucleic acids obtained or amplified from a cell), and detecting binding of the nucleic acid mixture to the array to produce an expression profile. Alternatively, the expression profile can be determined using a method and/or apparatus that does not require an array, such as SAGE or quantitative PCR with multiple primers.

A reference expression profile can be a profile including one or more of: (1) a profile from a sample from a normal subject, (2) a profile from a sample suspected of having or having a disorder, e.g., a neoplastic disorder, or metastatic disorder; (3) a profile from a sample from a subject having a metastatic disorder and undergoing treatment; and (4) a profile from a sample from a subject being evaluated, e.g., an earlier sample or a normal sample of the same subject. For example, the reference expression profile can be the profile of a cancerous cell line, e.g., a metastatic cancer cell line, e.g., a lung adenocarcinoma cell line, e.g., a lung adenocarcinoma cell line described herein.

A reference expression profile can also be an expression profile obtained from any suitable standard, e.g., a nucleic acid mixture. A sample expression profile is compared to a reference expression profile to produce a difference profile. Preferably, the sample expression profile is compared indirectly to the reference profile. For example, the sample expression profile is compared in multi-dimensional space to a cluster of reference expression profiles.

In still another aspect, the invention features an array. The array includes a substrate having a plurality of addresses. Each address of the plurality includes a capture probe, e.g., a unique capture probe. An address can have a single species of capture probe, e.g., each address recognizes a single species (e.g., a nucleic acid or polypeptide species). The addresses can be disposed on the substrate in a two-dimensional or three-dimensional configuration. At least one address of the plurality includes a capture probe such as an antibody or an antibody derivative that hybridizes specifically to a nucleic acid selected from Group I, II, III, or IV. A plurality of addresses can include addresses having nucleic acid capture probes for all the nucleic acids of Group I, II, III, and/or IV (i.e., 100% of the nucleic acids), or a fraction of the nucleic acids of Group I, II, III, and/or IV, e.g., at least 20%, 40%, 50%, 60%, 80%, or 90% of the nucleic acids of Group I, II, III, and/or IV. Alternatively, at least one address of the plurality includes a capture probe that binds specifically to a polypeptide selected from the group of polypeptides encoded by the nucleic acids of Group I, II, III, and/or IV. In some embodiments, the plurality of addresses includes addresses having polypeptide capture probes for all the nucleic acids of Group I, II, III, and/or IV (i.e., 100% of the nucleic acids) or a fraction of the nucleic acids of Group I, II, III, and/or IV, e.g., at least 20%, 40%, 50%, 60%, 80%, or 90% of the nucleic acids of Group I, II, III, and/or IV. An array can have a density consisting of at least 10, 50, 100, 200, 500, 10³, 10⁴, 10⁵, 10⁶, 10⁷, 10⁸, or 10⁹ or more addresses per cm² and ranges between.

In further another aspect, the invention features a set of probes that hybridize to a set of nucleic acids selected from Group I, II, III, and/or IV. The set of probes can include at least 2, 5, 10, 20, 30, 50 or more nucleic acids. The probes can be perfectly matched probes to the nucleic acids selected from Group I, II, III, and/or IV. However, mismatch probes having less than 5% mismatched nucleotides can also be used. The probes may be attached to a polymer, soluble or insoluble, naturally occurring or synthetic. The probes may be attached to a solid support (e.g., a planar array), in a gel matrix, or in solution.

Also featured is a method for diagnosis that includes providing a sample from a subject; determining the sample expression profile; comparing the sample expression profile to a reference expression profile, wherein the profile includes one or more values representing the levels of expression of one or more nucleic acids selected from Group I, II, III, and/or IV; and categorizing the subject as having tumor invasive potential or metastatic development when the sample expression profile is found to be altered from the reference expression profile. In some embodiments, the expression profile includes multiple values for the levels of expression of nucleic acids from Group I, II, III, and/or IV, e.g., all the nucleic acids of Group I, II, III, and/or IV (i.e., 100% of the nucleic acids), or a fraction of the nucleic acids of Group I, II, III, and/or IV, e.g., at least 20%, 40%, 50%, 60%, 80%, or 90% of the nucleic acids of Group I, II, III, and/or IV. The method can further include comparing the value or the profile (i.e., multiple values) to a reference value or a reference profile.

In some other embodiments, the reference profile is the profile of a cell line derived from a cancer, e.g., a metastatic cell line serially passaged. Examples of the cell lines are human lung adenocarcinoma cell lines of different invasive and metastatic capacities, e.g., CL₁₋₀ and its sublines (e.g., CL₁₋₁ and CL₁₋₅).

An alteration in the expression of one or more nucleic acids of the profile is an indication that the subject has or is disposed to having tumor invasive potential or metastatic development. Preferably, expression of a plurality of nucleic acids of the profile (e.g., at least about 5%, 10%, 15%, 20%, 40%, 50%, 60%, 70%, 80%, or 90%) is altered. The expression or the expression profile can be obtained from an array by the method described herein, or from a method and/or apparatus that does not require an array, such as SAGE or quantitative PCR with multiple primers. Alternatively, the expression or the expression profile can be determined by any combination of the just described methods.

In addition to diagnosing tumor invasive potential or metastatic development in a sample, this method can also be used to (1) monitor a subject during tumor treatment; or (2) monitor a treatment for tumor metastasis in a subject.

The subject expression profile can be determined in a subject during treatment. The subject expression profile can be compared to a reference profile or to a profile obtained from the subject prior to treatment or prior to onset of the disorder. In a preferred embodiment, the subject expression profile is determined at intervals (e.g., regular intervals) during treatment.

Still also featured is a method for screening for a test compound useful in the prevention or treatment of tumor metastasis. The method includes: providing one or more reference expression profiles; contacting the compound to a cell; determining a compound-associated expression profile, e.g., using a method described herein; and comparing the compound-associated expression profile to at least one reference profile. The compound-associated expression profile and the reference profile, including a subject expression profile and the reference profile, include one or more values representing the level of expression of one or more nucleic acids selected from Group I, II, III, and/or IV. In some embodiments, the profiles include multiple values for the level of expression of nucleic acids from Group I, II, III, and/or IV, e.g., all the nucleic acids of Group I, II, III, and/or IV (i.e., 100% of the nucleic acids) or a fraction of the nucleic acids of Group I, II, III, and/or IV, e.g., at least 20%, 40%, 50%, 60%, 80%, or 90% of the nucleic acids of Group I, II, III, and/or IV. In some other embodiments, the profile is the profile of at least two cell lines which are clonally related. Examples of the cell lines are human lung adenocarcinoma cell lines of different invasive and metastatic capacities, e.g., CL₁₋₀ and its sublines (e.g., CL₁₋₁ and CL₁₋₅).

This method for screening may also include comparing the compound-associated expression profile to a plurality of reference profiles (e.g., all reference profiles), and identifying a most similar reference profile as an indication of the efficacy and/or utility of the compound. Multiple compound-associated expression profiles can be determined at periodic intervals after contact with the agent. The expression profile can be obtained from an array by the method described herein, or from a method and/or apparatus that does not require an array, such as SAGE or quantitative PCR with multiple primers. Alternatively, the expression profile can be determined by any combination of the just described methods.

This invention also features cell lines that are clonally related and have different invasion capabilities, such as human lung adenocarcinoma cell lines, e.g., CL₁₋₀ and its sublines (e.g., CL₁₋₁ and CL₁₋₅). The cell lines can be used to determine a sample expression value or expression profile or a reference expression value or expression profile.

This invention still also features a transactional method of evaluating a subject. The method includes: (1) obtaining a sample from a caregiver; (2) determining a sample expression profile for the sample; and (3) transmitting a result to the caregiver. Optionally, the method further includes either or both of steps: (4) comparing the sample expression profile to one or more reference expression profiles; and (5) selecting the reference expression profile most similar to the subject expression profile. The reference expression profiles can be include one or more of: (1) a profile from a sample from a normal subject; (2) a profile from a sample suspected of having or having a disorder, e.g., a neoplastic disorder, or metastatic disorder; (3) a profile from a sample from a subject having a metastatic disorder and undergoing a treatment; and (4) a profile from the subject being evaluated, e.g., an earlier profile or a normal profile of the same subject. The subject expression profile and the reference profiles include one or more values representing the level of expression of one or more nucleic acids selected from Group I, II, III, and/or IV, e.g., all the nucleic acids of Group I, II, III, and/or IV (i.e., 100% of the nucleic acids) or a fraction of the nucleic acids of Group I, II, III, and/or IV, e.g., at least 20%, 40%, 50%, 60%, 80%, or 90% of the nucleic acids of Group I, II, III, and/or IV.

The result transmitted to the caregiver can be one or more of: information about the subject expression profile, e.g., raw or processed expression profile data and/or a graphical representation of the profile; a difference expression profile obtained by comparing the subject expression profile to a reference profile; a descriptor of the most similar reference profile; the most similar reference profile; and a diagnosis or treatment associated with the most similar reference profile. The result can be transmitted across a computer network, e.g., the result can be in the form of a computer transmission (e.g., across the Internet or a private network, e.g., a virtual private network). The result can be transmitted across a telecommunications network, e.g., using a telephone or mobile phone. The results can compressed and/or encrypted.

In the context of expression profiles herein, "most similar" refers to a profile, which for more than one value of the profile, compares favorably to a given profile. A variety of routine statistical measures can be used to compare two reference profiles. One possible metric is the length (i.e. Euclidean distance) of a difference vector representing the difference between the two profiles. Each of the subject and reference profile is represented as a multi-dimensional vector, wherein the coordinate of each dimension is a value in the profile. The distance of the difference vector is calculated using standard vectorial mathematics. In another embodiment, values for different nucleic acids in the profile are weighted for comparison.

Also within the scope of this invention is a computer medium having encoded thereon computer-readable instructions to effect the following steps: receive a subject expression profile; access a database of reference expression profiles; and either (1) select a matching reference profile most similar to the subject expression profile or (2) determine at least one comparison score for the similarity of the subject expression profile to at least one reference profile. The subject expression profile and the reference profiles include one or more values representing the levels of expression of one or more nucleic acids selected from Group I, II, III, and/or IV. In a preferred embodiment, the profiles include multiple values for the levels of expression of nucleic acids from Group I, II, III, and/or IV, e.g., all the nucleic acids of Group I, II, III, and/or IV (i.e., 100% of the nucleic acids) or a fraction of the nucleic acids of Group I, II, III, and/or IV, e.g., at least 20%, 40%, 50%, 60%, 80%, or 90% of the nucleic acids of Group I, II, III, and/or IV.

The instructions may further include instructions to create a graphical user interface that can display a sample expression profile and/or a reference profile. For example, a subset of or all values of the profile can be depicted as a graphic having a color dependent on the magnitude of the value. The graphical user interface can also allow the user to select a reference profile from a plurality of reference profiles, and can depict a comparison between the sample expression profile and the selected reference profile. The computer medium can further include, e.g., have encoded thereon, data records for one or more reference profiles.

This invention also features a computer medium having a plurality of digitally encoded data records. Each data record includes values representing the levels of expression of one or more nucleic acids selected from Group I, II, III, and/or IV, and a descriptor of the sample. The values may include multiple values for the level of expression of nucleic acids from Group I, II, III, and/or IV, e.g., all the nucleic acids of Group I, II, III, and/or IV (i.e., 100% of the nucleic acids) or a fraction of the nucleic acids of Group I, II, III, and/or IV, e.g., at least 20%, 40%, 50%, 60%, 80%, or 90% of the nucleic acids of Group I, II, III, and/or IV. The descriptor of the sample can be an identifier of the sample, a subject from which the sample was derived (e.g., a patient), a diagnosis (e.g., a metastasis disorder), or a treatment (e.g., a preferred treatment).

In one embodiment, the data records include records for one or more samples from a normal individual, an abnormal individual (e.g., an individual having a metastasis disorder), and an abnormal individual under a treatment. The data record may further include a value representing the level of expression for each nucleic acid detected by a capture probe on an array described herein.

The details of one or more embodiments of the invention are set forth in the description below. Other features, objects, and advantages of the invention will be apparent from the description and from the claims.

**Table I**

| (Group I): | | | |
|---|---|---|---|
| Unigene Cluster | IMAGE ID | Genbank Accession no. | Putative gene name |
| Hs.75878 | 23633 | R39603 | early development regulator 2 (homolog of polyhomeotic 2) |
| Hs.81071 | 229703 | H66472 | extracellular matrix protein 1 |
| Hs.89563 | 148578 | H12586 | nuclear cap binding protein 1, 80kD |
| Hs.202949 | 24829 | R38857 | KIAA1102 protein |
| Hs.635 | 300048 | N78918 | calcium channel, voltage-dependent, beta 1 subunit |
| Hs.79274 | 344912 | W72244 | annexin A5 |
| Hs.263435 | 345832 | W72690 | histidine ammonia-lyase |
| Hs.278593 | 530715 | AA069915 | interleukin 18 binding protein |
| Hs.19131 | 307932 | N93047 | transcription factor Dp-2 (E2F dimerization partner 2) |
| Hs.8117 | 108245 | T69807 | erbb2-interacting protein ERBIN |
| Hs.1501 | 114116 | T79471 | syndecan 2 (heparan sulfate proteoglycan 1, cell surface-associated, fibroglycan) |
| Hs.103042 | 174835 | H30016 | microtubule-associated protein 1B |
| Hs.77313 | 310428 | N98775 | cyclin-dependent kinase (CDC2-like) 10 |
| Hs.23617 | 44582 | H07076 | hypothetical protein FLJ20531 |
| Hs.153 | 549076 | AA083222 | ribosomal protein L7 |
| Hs.8123 | 322820 | W15314 | chromobox homolog 3 (Drosophila HP1 gamma) |
| Hs.75716 | 323255 | W42998 | plasminogen activator inhibitor, type II (arginine-serpin) |
| Hs.77326 | 323605 | W44341 | insulin-like growth factor binding protein 3 |
| Hs.75621 | 200421 | R97243 | protease inhibitor 1 (anti-elastase), alpha-1-antitrypsin |
| Hs.99910 | 202674 | H53527 | phosphofructokinase, platelet |
| Hs.24340 | 131368 | R23188 | centaurin beta2 |
| Hs.1770 | 203816 | H56135 | ligase I, DNA, ATP-dependent |
| Hs.150423 | 324698 | W47128 | cyclin-dependent kinase 9 (CDC2-related kinase) |
| Hs.145956 | 429284 | AA007349 | zinc finger protein 226 |
| Hs.265829 | 44280 | H06518 | integrin, alpha 3 (antigen CD49C, alpha 3 subunit of VLA-3 receptor) |
| Hs.3068 | 38359 | R49463 | SWI/SNF related, matrix associated, actin dependent regulator of chromatin, subfamily a, member 3 |
| Hs.103106 | 340731 | W56305 | Homo sapiens mRNA for G7b protein (G7b gene, located in the class III region of the major histocompatibility complex |
| Hs.83341 | 49318 | H15718 | AXL receptor tyrosine kinase |
| Hs.51147 | 221208 | H91843 | guanine nucleotide binding protein (G protein), alpha transducing activity polypeptide 1 |
| Hs.74579 | 52262 | H22893 | KIAA0263 gene product |
| Hs.250687 | 294499 | N69526 | transient receptor potential channel 1 |
| Hs.202379 | 471256 | AA034479 | meiotic recombination (S. cerevisiae) 11 homolog B |
| Hs.86386 | 21555 | T65116 | myeloid cell leukemia sequence 1 (BCL2-related) |
| Hs.20991 | 25755 | R37131 | SET domain, bifurcated, 1 |
| Hs.85302 | 67051 | T70335 | adenosine deaminase, RNA-specific, B1 (homolog of rat RED1) |
| Hs.78473 | 308063 | N95309 | N-deacetylase/N-sulfotransferase (heparan glucosaminyl) 2 |
| Hs.102 | 346840 | W79815 | aminomethyltransferase (glycine cleavage system protein T) |
| Hs.75516 | 109211 | T81117 | tyrosine kinase 2 |
| Hs.73769 | 308366 | N93766 | folate receptor 1 (adult) |
| Hs.14376 | 110760 | T90619 | actin, gamma 1 |
| Hs.15154 | 110740 | T90558 | sushi-repeat-containing protein, X chromosome |
| Hs.83727 | 309032 | N92864 | cleavage and polyadenylation specific factor 1, 160kD subunit |
| Hs.69771 | 544635 | AA075297 | B-factor, properdin |
| Hs.12163 | 544672 | AA074799 | eukaryotic translation initiation factor 2, subunit 2 (beta, 38kD) |
| Hs.211539 | 166201 | R87490 | eukaryotic translation initiation factor 2, subunit 3 (gamma, 52kD) |
| Hs.80315 | 179283 | H50251 | SH3-domain GRB2-like 3 |
| Hs.17704 | 120850 | T95879 | PERB11 family member in MHC class I region |
| Hs.63489 | 365849 | AA025527 | protein tyrosine phosphatase, non-receptor type 6 |
| Hs.80776 | 192966 | H41410 | phospholipase C, delta 1 |
| Hs.180842 | 323468 | W45605 | ribosomal protein L13 |
| Hs.21022 | 47510 | H11603 | adaptor-related protein complex 3, beta 2 subunit |
| Hs.270845 | 50080 | H17934 | kinesin-like 5 (mitotic kinesin-like protein 1) |
| Hs.78902 | 52494 | H23010 | voltage-dependent anion channel 2 |
| Hs.85296 | 484874 | AA037229 | Integrin beta 3 {alternatively spliced, clone beta 3C} [human, erythroleukemia cell HEL, mRNA Partial, 409 nt] |
| Hs.75535 | 300051 | N78927 | myosin, light polypeptide 2, regulatory, cardiac, slow |
| Hs.80680 | 485005 | AA037708 | major vault protein |
| Hs.82071 | 343685 | W69165 | Cbp/p300-interacting transactivator, with Glu/Asp-rich carboxy-terminal domain, 2 |
| Hs.78465 | 40991 | R56065 | v-jun avian sarcoma virus 17 oncogene homolog |
| Hs.99914 | 347097 | W79487 | ribosomal protein L22 |
| Hs.73818 | 489338 | AA058525 | ubiquinol-cytochrome c reductase hinge protein |
| Hs.79889 | 44722 | H06712 | monocyte to macrophage differentiation-associated |
| Hs.92198 | 257128 | N26830 | calcium-regulated heat-stable protein (24kD) |
| Hs.179898 | 321445 | W32294 | HSPC055 protein |
| Hs.18268 | 46408 | H09257 | adenylate kinase 5 |
| Hs.114346 | 567001 | AA152406 | cytochrome c oxidase subunit VIIa polypeptide 1 (muscle) |
| Hs.155342 | 47306 | H11054 | protein kinase C, delta |
| Hs.2961 | 377441 | AA055242 | S100 calcium-binding protein A3 |
| Hs.7994 | 33398 | R44070 | hypothetical protein FLJ20551 |
| Hs.180069 | 591311 | AA159002 | nuclear respiratory factor 1 |
| Hs.76753 | 47648 | H16257 | endoglin (Osler-Rendu-Weber syndrome 1) |
| Hs.279607 | 592815 | AA158262 | calpastatin |
| Hs.182979 | 323915 | W46302 | ribosomal protein L12 |
| Hs.118442 | 595244 | AA164211 | cyclin C |
| Hs.75139 | 49363 | H16576 | partner of RAC1 (arfaptin 2) |
| Hs.76240 | 324895 | W49669 | adenylate kinase 1 |
| Hs.74578 | 416266 | W86149 | DEAD/H (Asp-Glu-Ala-Asp/His) box polypeptide 9 (RNA helicase A, nuclear DNA helicase II; leukophysin) |
| Hs.259776 | 416764 | W86580 | Human 3-hydroxyacyl-CoA dehydrogenase, isoform 2 mRNA, complete cds |
| Hs.14331 | 327145 | W02662 | S100 calcium-binding protein A13 |
| Hs.181289 | 328416 | W38424 | elastase 3, pancreatic (protease E) |
| Hs.173554 | 341640 | W58185 | ubiquinol-cytochrome c reductase core protein II |
| Hs.267819 | 223243 | H85614 | protein phosphatase 1, regulatory (inhibitor) subunit 2 |
| Hs.83765 | 52414 | H24390 | dihydrofolate reductase |
| Hs.173205 | 25760 | R37231 | nucleophosmin (nucleolar phosphoprotein B23, numatrin) |
| Hs.2730 | 306373 | N90702 | heterogeneous nuclear ribonucleoprotein L |
| Hs.183994 | 307127 | N91788 | protein phosphatase 1, catalytic subunit, alpha isoform |
| Hs.50889 | 307055 | N89677 | (clone PWHLC2-24) myosin light chain 2 |
| Hs.211973 | 109285 | T80836 | homolog of Yeast RRP4 (ribosomal RNA processing 4), 3'-5'-exoribonuclease |
| Hs.184776 | 243274 | H94695 | ribosomal protein L23a |
| Hs.2175 | 246180 | N55510 | colony stimulating factor 3 receptor (granulocyte) |
| Hs.180946 | 544885 | AA075385 | ribosomal protein L5 |
| Hs.32952 | 323834 | W46372 | keratin, hair, basic, 1 |
| Hs.157777 | 202915 | H54091 | Homo sapiens mRNA; cDNA DKFZp434G0118 (from clone DKFZp434G0118) |
| Hs.80288 | 35091 | R43793 | heat shock 70kD protein-like 1 |
| Hs.132243 | 278624 | N66195 | aminopeptidase puromycin sensitive |
| Hs.75607 | 470841 | AA031774 | myristoylated alanine-rich protein kinase C substrate (MARCKS, 80K-L) |
| Hs.99858 | 233358 | H79784 | ribosomal protein L7a |
| Hs.82163 | 26094 | R37282 | monoamine oxidase B |
| Hs.82128 | 155195 | R70262 | 5T4 oncofetal trophoblast glycoprotein |
| Hs.146550 | 42511 | R60961 | myosin, heavy polypeptide 9, non-muscle |
| Hs.75334 | 27524 | R39966 | exostoses (multiple) 2 |
| Hs.85087 | 166004 | R87406 | latent transforming growth factor beta binding protein 4 |
| Hs.198443 | 43783 | H05672 | inositol 1,4,5-triphosphate receptor, type 1 |
| Hs.64639 | 310347 | N98760 | glioma pathogenesis-related protein |
| Hs.243987 | 124194 | R01769 | GATA-binding protein 4 |
| Hs.6980 | 322522 | W15247 | aldo-keto reductase family 7, member A2 (aflatoxin aldehyde reductase) |
| Hs.167531 | 195423 | R89611 | Homo sapiens mRNA full length insert cDNA clone EUROIMAGE 195423 |
| Hs.5855 | 46889 | H10154 | Homo sapiens mRNA; cDNA DKFZp434D0818 (from clone DKFZp434D0818) |
| Hs.418 | 323181 | W42634 | fibroblast activation protein, alpha; seprase |
| Hs.182418 | 33899 | R44535 | endonuclease G |
| Hs.150555 | 593061 | AA158728 | protein predicted by clone 23733 |
| Hs.180577 | 133172 | R26450 | granulin |
| Hs.286 | 272457 | N35801 | ribosomal protein L4 |
| Hs.75428 | 275935 | R93344 | superoxide dismutase 1, soluble (amyotrophic lateral sclerosis 1 (adult)) |
| Hs.223241 | 50603 | H17646 | eukaryotic translation elongation factor 1 delta (guanine nucleotide exchange protein) |
| Hs.95011 | 428054 | AA002062 | syntrophin, beta 1 (dystrophin-associated protein A1, 59kD, basic component 1) |
| Hs.75772 | 38329 | R49540 | nuclear receptor subfamily 3, group C, member 1 |
| Hs.242463 | 510899 | AA099945 | keratin 8 |
| Hs.27747 | 52375 | H22978 | G protein-coupled receptor 37 (endothelin receptor type B-like) |
| Hs.65424 | 297795 | N69950 | tetranectin (plasminogen-binding protein) |
| Hs.82906 | 129381 | R11676 | cell division cycle 20, S.cerevisiae homolog |
| Hs.23119 | 485665 | AA041521 | ITBA1 gene |
| Hs.85570 | 344436 | W73253 | hypothetical protein similar to beta-transducin family |
| Hs.44499 | 344704 | W73034 | small EDRK-rich factor 2 |
| Hs.274416 | 306510 | N91803 | NADH dehydrogenase (ubiquinone) 1 alpha subcomplex, 6 (14kD, B14) |
| Hs.78547 | 345201 | W72342 | Human mRNA for zinc finger protein (clone 647) |
| Hs.151706 | 154045 | R48810 | KIAA0134 gene product |
| Hs.7644 | 346040 | W72152 | H1 histone family, member 2 |
| Hs.9242 | 346109 | W72744 | purine-rich element binding protein B |
| Hs.1076 | 346130 | W72751 | small proline-rich protein 1B (cornifin) |
| Hs.46405 | 346780 | W78101 | polymerase (RNA) II (DNA directed) polypeptide F |
| Hs.197728 | 155738 | R72084 | carboxylesterase 2 (intestine, liver) |
| Hs.84318 | 308501 | N95782 | replication protein A1 (70kD) |
| Hs.99969 | 347175 | W80463 | fusion, derived from t(12;16) malignant liposarcoma |
| Hs.170027 | 243603 | N49725 | mouse double minute 2, human homolog of; p53-binding protein |
| Hs.78888 | 308755 | N9521 | diazepam binding inhibitor (GABA receptor modulator, acyl-Coenzyme A binding protein) |
| Hs.258730 | 246201 | N55530 | heme-regulated initiation factor 2-alpha kinase |
| Hs.180946 | 544915 | AA075496 | ribosomal protein L5 |
| Hs.88411 | 113635 | T79220 | DNA segment on chromosome 6 (unique) 49 expressed sequence |
| Hs.3281 | 172857 | H20073 | neuronal pentraxin II |
| Hs.256697 | 174619 | H27885 | histidine triad nucleotide-binding protein |
| Hs.13456 | 310457 | N99978 | Homo sapiens clone 24747 mRNA sequence |
| Hs.15196 | 510032 | AA053393 | putative receptor protein |
| Hs.7736 | 31276 | R42876 | hypothetical protein |
| Hs.100724 | 510275 | AA053166 | peroxisome proliferative activated receptor, gamma |
| Hs.16059 | 366695 | AA029690 | HSPC009 protein |
| Hs.81469 | 376052 | AA039353 | nucleotide binding protein 1 (E.coli MinD like) |
| Hs.80684 | 267145 | N23950 | high-mobility group (nonhistone chromosomal) protein 2 |
| Hs.118786 | 86004 | T62812 | metallothionein 2A |
| Hs.82251 | 469748 | AA028056 | myosin IC |
| Hs.65114 | 511604 | AA115797 | keratin 18 |
| Hs.8383 | 222735 | H86444 | bromodomain adjacent to zinc finger domain, 2B |
| Hs.11638 | 526088 | AA076383 | FACL5 for fatty acid coenzyme A ligase 5 |
| Hs.50130 | 343578 | W69632 | necdin (mouse) homolog |
| Hs.2083 | 485849 | AA040427 | CDC-like kinase 1 |
| Hs.180532 | 26483 | R39784 | heat shock 90kD protein 1, alpha |
| Hs.75323 | 42313 | R60946 | prohibitin |
| Hs.50640 | 306873 | N91935 | JAK binding protein |
| Hs.77031 | 43297 | H05065 | Sp2 transcription factor |
| Hs.64593 | 309391 | N94313 | ATP synthase, H+ transporting, mitochondrial F1F0, subunit d |
| Hs.135281 | 43567 | H05938 | alpha-actinin-2-associated LIM protein |
| Hs.1239 | 309853 | N94637 | alanyl (membrane) aminopeptidase (aminopeptidase N, aminopeptidase M, microsomal aminopeptidase, CD13, p150) |
| Hs.79069 | 21505 | T65541 | cyclin G2 |
| Hs.169449 | 30209 | R16311 | protein kinase C, alpha |
| Hs.77628 | 31155 | R42594 | steroidogenic acute regulatory protein related |
| Hs.7644 | 257094 | N30791 | H1 histone family, member 2 |
| Hs.3254 | 259977 | N32606 | ribosomal protein L23-like |
| Hs.82767 | 563608 | AA100423 | sperm specific antigen 2 |
| Hs.65114 | 563957 | AA101381 | keratin 18 |
| Hs.118893 | 563884 | AA101407 | p53-responsive gene 2 |
| Hs.77221 | 46367 | H09959 | choline kinase |
| Hs.156346 | 46597 | H09978 | topoisomerase (DNA) II alpha (170kD) |
| Hs.274364 | 712871 | AA281981 | 6-phosphofructo-2-kinase/fructose-2,6-biphosphatase 3 |
| Hs.42484 | 567179 | AA148598 | hypothetical protein FLJ10618 |
| Hs.172665 | 47384 | H10779 | methylenetetrahydrofolate dehydrogenase (NADP+ dependent), methenyltetrahydrofolate cyclohydrolase, formyltetrahydrofolate s |
| Hs.35384 | 198546 | R94868 | ring finger protein 1 |
| Hs.180655 | 205953 | H58497 | serine/threonine kinase 12 |
| Hs.83173 | 327182 | W02748 | cyclin D3 |
| Hs.75199 | 37209 | R50927 | protein phosphatase 2, regulatory subunit B (B56), beta isoform |
| Hs.241515 | 37394 | R49679 | COX11 (yeast) homolog, cytochrome c oxidase assembly protein |
| Hs.28777 | 283919 | N50797 | H2A histone family, member L |
| Hs.75752 | 285455 | N63245 | cytochrome c oxidase subunit VIIb |
| Hs.179735 | 340781 | W56747 | ras homolog gene family, member C |
| Hs.16364 | 38571 | R51498 | hypothetical protein FU10955 |
| Hs.77274 | 143356 | R74194 | plasminogen activator, urokinase |
| Hs.86386 | 146164 | R79099 | myeloid cell leukemia sequence 1 (BCL2-related) |
| Hs.155103 | 222259 | H83844 | eukaryotic translation initiation factor 1A, Y chromosome |
| Hs.75478 | 342260 | W61185 | KIAA0956 protein |
| Hs.82316 | 470583 | AA031928 | interferon-induced, hepatitis C-associated microtubular aggregate protein (44kD) |
| Hs.75428 | 39993 | R52548 | superoxide dismutase 1, soluble (amyotrophic lateral sclerosis 1 (adult)) |
| Hs.94234 | 297884 | N68934 | frizzled (Drosophila) homolog 1 |
| Hs.11899 | 21759 | T65127 | 3-hydroxy-3-methylglutaryl-Coenzyme A reductase |
| Hs.76152 | 343315 | W68075 | decorin |
| Hs.165843 | 344021 | W70096 | casein kinase 2, beta polypeptide |
| Hs.77356 | 25291 | R17745 | transferrin receptor (p90, CD71) |
| Hs.75232 | 40704 | R55993 | SEC14 (S. cerevisiae)-like 1 |
| Hs.2393 | 306175 | N90546 | phosphorylase kinase, alpha 1 (muscle) |
| Hs.8768 | 41144 | R59152 | hypothetical protein FLJ10849 |
| Hs.84981 | 21640 | T65431 | X-ray repair complementing defective |
| | | | repair in Chinese hamster cells 5 (double- |
| | | | strand-break rejoining; Ku autoantigen, 80kD) |
| Hs.77550 | 307169 | N91798 | CDC28 protein kinase 1 |
| Hs.227949 | 345522 | W72422 | SEC13 (S. cerevisiae)-like 1 |
| Hs.181028 | 307509 | N95162 | cytochrome c oxidase subunit Va |
| Hs.85838 | 156040 | R72509 | solute carrier family 16 (monocarboxylic acid transporters), member 3 |
| Hs.181165 | 307988 | N95281 | eukaryotic translation elongation factor 1 alpha 1 |
| Hs.108854 | 308000 | N92290 | HSPC163 protein |
| Hs.78060 | 42453 | R61298 | phosphorylase kinase, beta |
| Hs.26700 | 42358 | R59858 | Homo sapiens cDNA FLJ10309 fis, clone NT2RM2000287 |
| Hs.75103 | 27100 | R37146 | tyrosine 3-monooxygenase/tryptophan 5-monooxygenase activation protein, zeta polypeptide |
| Hs.16003 | 42714 | R60888 | retinoblastoma-binding protein 4 |
| Hs.119387 | 110774 | T90622 | KIAA0792 gene product |
| Hs.77890 | 357308 | W93728 | guanylate cyclase 1, soluble, beta 3 |
| Hs.250867 | 160363 | H22334 | zona pellucida glycoprotein 3A (sperm receptor) |
| Hs.180450 | 309185 | N99249 | ribosomal protein S24 |
| Hs.118131 | 309218 | N93850 | 5,10-methenyltetrahydrofolate synthetase (5-formyltetrahydrofolate cyclo-ligase) |
| Hs.117729 | 162681 | H28224 | keratin 14 (epidermolysis bullosa simplex, Dowling-Meara, Koebner) |
| Hs.195851 | 247237 | N57938 | actin, alpha 2, smooth muscle, aorta |
| Hs.78854 | 166145 | R87471 | ATPase, Na+/K+ transporting, beta 2 polypeptide |
| Hs.41688 | 250069 | H97140 | dual specificity phosphatase 8 |
| Hs.77256 | 121554 | T97906 | enhancer of zeste (Drosophila) homolog 2 |
| Hs.181163 | 45188 | H08826 | high-mobility group (nonhistone chromosomal) protein 17 |
| Hs.75319 | 510231 | AA053076 | ribonucleotide reductase M2 polypeptide |
| Hs.149436 | 563057 | AA113038 | kinesin family member SB |
| Hs.2934 | 46623 | H10294 | ribonucleotide reductase M1 polypeptide |
| Hs.79914 | 196071 | R89380 | lumican |
| Hs.79037 | 567278 | AA130632 | heat shock 60kD protein 1 (chaperonin) |
| Hs.180946 | 590007 | AA155824 | ribosomal protein L5 |
| Hs.85844 | 323685 | W44537 | neurotrophic tyrosine kinase, receptor, type 1 |
| Hs.26322 | 33430 | R43993 | cell cycle related kinase |
| Hs.6517 | 47514 | H12216 | amiloride-sensitive cation channel 1, neuronal (degenerin) |
| Hs.4209 | 323859 | W46361 | ribosomal protein, mitochondrial, L2 |
| Hs.831 | 37162 | R49317 | 3-hydroxymethyl-3-methylglutaryl-Coenzyme A lyase (hydroxymethylglutaricaciduria) |
| Hs.75862 | 23275 | R39273 | MAD (mothers against decapentaplegic, |
| | | | Drosophila) homolog 4 |
| Hs.117816 | 37540 | R50950 | sorcin |
| Hs.198287 | 139392 | R65579 | pregnancy specific beta-1-glycoprotein 11 |
| Hs.79117 | 38183 | R49222 | corticotropin releasing hormone receptor 1 |
| Hs.259842 | 37296 | R51092 | H91620p protein |
| Hs.232068 | 42396 | R60877 | transcription factor 8 (represses interleukin 2 expression) |
| Hs.274344 | 118942 | T92945 | hypothetical protein |
| Hs.234799 | 51558 | H21053 | breakpoint cluster region |
| Hs.98493 | 23330 | R38269 | X-ray repair complementing defective repair in Chinese hamster cells 1 |
| Hs.225841 | 23760 | R38172 | DKFZP434D193 protein |
| Hs.91985 | 52482 | H22986 | wingless-type MMTV integration site family, member 10B |
| Hs.173664 | 147016 | R80150 | v-erb-b2 avian erythroblastic leukemia viral oncogene homolog 2 (neuro/glioblastoma derived oncogene homolog) |
| Hs.179566 | 53273 | R16162 | Human clone 23799 mRNA sequence |
| Hs.180455 | 26222 | R37437 | RAD23 (S. cerevisiae) homolog A |
| Hs.77225 | 26790 | R37680 | ADP-ribosyltransferase (NAD+; poly (ADP-ribose) polymerase)-like 1 |
| Hs.63908 | 43168 | R60538 | heme oxygenase (decycling) 2 |
| Hs.3843 | 28140 | R40503 | dual specificity phosphatase 7 |
| Hs.89529 | 501617 | AA129588 | aldo-keto reductase family 1, member A1 (aldehyde reductase) |
| Hs.108043 | 36987 | R48940 | Friend leukemia virus integration 1 |
| Hs.174007 | 133122 | R26243 | von Hippel-Lindau syndrome |
| Hs.108110 | 546454 | AA081374 | DKFZP547E2110 protein |
| Hs.1908 | 310779 | W19210 | proteoglycan 1, secretory granule |
| Hs.268149 | 30747 | R42072 | putative methyltransferase |
| Hs.12743 | 363489 | AA019803 | Homo sapiens clone 24511 mRNA sequence |
| Hs.180255 | 186767 | H50623 | major histocompatibility complex, class II, DR beta 1 |
| Hs.108323 | 31669 | R43030 | ubiquitin-conjugating enzyme E2E 2 (homologous to yeast UBC4/5) |
| Hs.48876 | 123355 | T99625 | farnesyl-diphosphate farnesyltransferase 1 |
| Hs.174905 | 47304 | H10460 | KIAA0033 protein |
| Hs.76716 | 127599 | R09178 | pre-alpha (globulin) inhibitor, H3 polypeptide |
| Hs.25615 | 380437 | AA054135 | CGI-119 protein |
| Hs.247565 | 380875 | AA056094 | rhodopsin (retinitis pigmentosa 4, autosomal dominant) |
| Hs.8551 | 34872 | R44450 | PRP4/STK/WD splicing factor |
| Hs.6289 | 35871 | R46034 | growth factor receptor-bound protein 2 |
| Hs.3631 | 49932 | H29009 | immunoglobulin (CD79A) binding protein 1 |
| Hs.8128 | 50624 | H17907 | phosphatidylserine decarboxylase |
| Hs.183 | 22411 | T82477 | Duffy blood group |
| Hs.92323 | 511428 | AA126009 | FXYD domain-containing ion transport regulator 3 |
| Hs.33287 | 145106 | R77291 | nuclear factor I/B |
| Hs.76293 | 512246 | AA057636 | thymosin, beta 10 |
| Hs.17775 | 342742 | W68632 | p75NTR-associated cell death executor; ovarian granulosa cell protein (13kD) |
| Hs.6673 | 52600 | H29041 | trinucleotide repeat containing 15 |
| Hs.168383 | 145112 | R77293 | intercellular adhesion molecule 1 (CD54), human rhinovirus receptor |
| Hs.1420 | 180447 | R84974 | fibroblast growth factor receptor 3 (achondroplasia, thanatophoric dwarfism) |
| Hs.19196 | 487778 | AA045185 | ubiquitin-conjugating enzyme HBUCE1 |
| Hs.110637 | 487978 | AA054590 | homeo box A10 |
| Hs.180919 | 240151 | H82442 | inhibitor of DNA binding 2, dominant negative helix-loop-helix protein |
| Hs.275374 | 491456 | AA150441 | aldo-keto reductase family 1, member C1 (dihydrodiol dehydrogenase 1; 20-alpha (3-alpha)-hydroxysteroid dehydrogenase) |
| Hs.28505 | 502133 | AA126984 | ubiquitin-conjugating enzyme E2H (homologous to yeast UBC8) |
| Hs.83760 | 359187 | AA010108 | troponin I, skeletal, fast |
| Hs.274434 | 309917 | N94503 | Homo sapiens cDNA FLJ11346 fis, clone PLACE1010900 |
| Hs.55498 | 503118 | AA151550 | geranylgeranyl diphosphate synthase 1 |
| Hs.22028 | 173777 | H23796 | SNARE protein |
| Hs.86958 | 359976 | AA063518 | interferon (alpha, beta and omega) receptor 2 |
| Hs.13684 | 29797 | R42224 | hypothetical protein FLJ10761 |
| Hs.198951 | 309864 | N94468 | jun B proto-oncogene |
| Hs.278626 | 505225 | AA142922 | Arg/Abl-interacting protein ArgBP2 |
| Hs.77768 | 44495 | H07123 | heat shock protein, neuronal DNAJ-like 1 |
| Hs.169921 | 548957 | AA115186 | general transcription factor II, i, pseudogene 1 |
| Hs.159608 | 362864 | AA019525 | aldehyde dehydrogenase 10 (fatty aldehyde dehydrogenase) |
| Hs.23205 | 31567 | R42860 | membrane protein, palmitoylated 2 (MAGUK p55 subfamily member 2) |
| Hs.279850 | 45476 | H08115 | CGI-50 protein |
| Hs.183800 | 69255 | T54339 | Ran GTPase activating protein 1 |
| Hs.169474 | 561918 | AA085589 | DKFZP586J0119 protein |
| Hs.182217 | 31798 | R43131 | succinate-CoA ligase, ADP-forming, beta subunit |
| Hs.231581 | 561922 | AA085678 | myosin, heavy polypeptide 1, skeletal muscle, adult |
| Hs.181696 | 364698 | AA024422 | zinc finger protein 255 |
| Hs.40193 | 123815 | R01452 | hypothetical protein KIAA1259 |
| Hs.108043 | 71822 | T52520 | Friend leukemia virus integration 1 |
| Hs.151461 | 667365 | AA228085 | embryonic ectoderm development protein |
| Hs.1384 | 126309 | R06411 | 0-6-methylguanine-DNA methyltransferase |
| Hs.19718 | 126379 | R06556 | protein tyrosine phosphatase, receptor type, U |
| Hs.46423 | 667303 | AA227555 | H4 histone family, member G |
| Hs.172458 | 47251 | H10977 | iduronate 2-sulfatase (Hunter syndrome) |
| Hs.250711 | 375752 | AA033816 | dipeptidyl carboxypeptidase 1 (angiotensin I converting enzyme) |
| Hs.102135 | 265879 | N21005 | signal sequence receptor, delta (translocon-associated protein delta) |
| Hs.121559 | 199577 | R96579 | CGI-30 protein |
| Hs.52763 | 66919 | T67474 | anaphase-promoting complex subunit 7 |
| Hs.22891 | 267666 | N23174 | solute carrier family 7 (cationic amino acid transporter, y+ system), member 8 |
| Hs.64025 | 132373 | R26526 | basonuclin |
| Hs.75253 | 206506 | H60000 | isocitrate dehydrogenase 3 (NAD+) gamma |
| Hs.90291 | 416587 | W86459 | laminin, beta 2 (laminin S) |
| Hs.9552 | 108323 | T70592 | binder of Arl Two |
| Hs.278736 | 53070 | R16049 | cell division cycle 42 (GTP-binding protein, 25kD) |
| Hs.66394 | 50188 | H17943 | ring forger protein 4 |
| Hs.5120 | 33297 | R43960 | dynein, cytoplasmic, light polypeptide |
| Hs.739 | 86044 | T62884 | 6-phosphofructo-2-kinase/fructose-2,6-biphosphatase 1 |
| Hs.151134 | 37433 | R50947 | oxidase (cytochrome c) assembly 1-like |
| Hs.82916 | 21912 | T65288 | chaperonin containing TCP1, subunit 6A (zeta 1) |
| Hs.17364 | 119345 | T94329 | zinc finger protein 79 (pT7) |
| Hs.73957 | 47559 | H11455 | RAB5A, member RAS oncogene family |
| Hs.155206 | 51480 | H24014 | serine/threonine kinase 25 (Ste20, yeast homolog) |
| Hs.89591 | 50182 | H17883 | Kallmann syndrome 1 sequence |
| Hs.267319 | 144767 | R77223 | endogenous retroviral protease |
| Hs.90093 | 39039 | R51827 | heat shock 70kD protein 4 |
| Hs.697 | 40017 | R52654 | cytochrome c-1 |
| Hs.170160 | 24743 | R37588 | RAB2, member RAS oncogene family-like |
| Hs.79530 | 21445 | T65107 | M5-14 protein |
| Hs.75925 | 24592 | R38826 | proteasome (prosome, macropain) inhibitor subunit 1 (PI31) |
| Hs.15020 | 25700 | R36870 | homolog of mouse quaking QKI (KH domain RNA binding protein) |
| Hs.180532 | 25792 | R36846 | heat shock 90kD protein 1, alpha |
| Hs.36587 | 156436 | R73564 | protein phosphatase 1, regulatory subunit 7 |
| Hs.111515 | 26964 | R37773 | CGI-43 protein |
| Hs.2838 | 42363 | R61319 | malic enzyme 3, NADP(+)-dependent, mitochondrial |
| Hs.19122 | 501557 | AA135645 | eukaryotic translation initiation factor 4E-like 3 |
| Hs.68318 | 547571 | AA083845 | hypothetical protein FLJ20344 |
| Hs.80475 | 45923 | H09542 | polymerase (RNA) II (DNA directed) polypeptide J (13.3kD) |
| Hs.30888 | 46147 | H09580 | cytochrome c oxidase subunit VIIa polypeptide 2 like |
| Hs.12887 | 47422 | H11255 | Soluble VEGF receptor |
| Hs.108931 | 377054 | AA057615 | MAGUK protein p55T; Protein Associated with Lins 2 |
| Hs.173936 | 202498 | H53121 | interleukin 10 receptor, beta |
| Hs.180669 | 50107 | H16738 | conserved gene amplified in osteosarcoma |
| Hs.1032 | 328897 | W45438 | regenerating islet-derived 1 alpha (pancreatic stone protein, pancreatic thread protein) |
| Hs.119597 | 214028 | H70783 | stearoyl-CoA desaturase (delta-9-desaturase) |
| Hs.117848 | 285437 | N66390 | hemoglobin, epsilon 1 |
| Hs.95665 | 40787 | R56077 | hypothetical protein |
| Hs.93304 | 238821 | H65030 | phospholipase A2, group VII (platelet-activating factor acetylhydrolase, plasma) |
| Hs.195464 | 489918 | AA114828 | filamin A, alpha (actin-binding protein-280) |
| Hs.169832 | 490387 | AA120779 | zinc finger protein 42 (myeloid-specific retinoic acid- responsive) |
| Hs.74471 | 309079 | N92894 | gap junction protein, alpha 1, 43kD (connexin 43) |
| Hs.83147 | 491560 | AA115549 | guanine nucleotide binding protein-like 1 |
| Hs.8248 | 28502 | R37489 | NADH dehydrogenase (ubiquinone) Fe-S protein 1 (75kD) (NADH-coenzyme Q reductase) |
| Hs.211584 | 28422 | R40649 | neurofilament, light polypeptide (68kD) |
| Hs.184050 | 28627 | R40473 | v-Ki-ras2 Kirsten rat sarcoma 2 viral oncogene homolog |
| Hs.79691 | 503974 | AA130144 | LIM domain protein |
| Hs.108139 | 504086 | AA131858 | zinc finger protein 212 |
| Hs.75280 | 29795 | R42222 | glycyl-tRNA synthetase |
| Hs.1857 | 363198 | AA018928 | phosphodiesterase 6G, cGMP-specific, rod, gamma |
| Hs.173063 | 68616 | T49793 | transducin-like enhancer of split 2, homolog of Drosophila E(sp1) |
| Hs.180370 | 123883 | R00785 | cofilin 1 (non-muscle) |
| Hs.31939 | 67185 | T52650 | manic fringe (Drosophila) homolog |
| Hs.167835 | 365363 | AA025214 | acyl-Coenzyme A oxidase |
| Hs.90093 | 128251 | R11513 | heat shock 70kD protein 4 |
| Hs.587 | 129068 | R10850 | arylacetamide deacetylase (esterase) |
| Hs.82254 | 48584 | H16096 | zuotin related factor 1 |
| Hs.167839 | 324749 | W47158 | KIAA0395 protein |
| Hs.152818 | 272871 | N36004 | ubiquitin specific protease 8 |
| Hs.18069 | 36128 | R62434 | protease, cysteine, 1 (legumain) |
| Hs.75485 | 21625 | T65577 | ornithine aminotransferase (gyrate atrophy) |
| Hs.83869 | 41134 | R58972 | hypothetical protein |
| Hs.4788 | 22490 | T87616 | KIAA0253 protein |
| Hs.37501 | 214636 | H73190 | MAD (mothers against decapentaplegic, Drosophila) homolog 5 |
| Hs.199429 | 51483 | H24016 | Homo sapiens mRNA; cDNA DKFZp434M2216 (from clone DKFZp434M2216) |
| Hs.166887 | 51949 | H24315 | copine I |
| Hs.93379 | 23234 | R38667 | eukaryotic translation initiation factor 4B |
| Hs.241543 | 39177 | R54415 | DKFZP586F1524 protein |
| Hs.25333 | 470402 | AA031292 | interleukin 1 receptor, type II |
| Hs.11223 | 525983 | AA076246 | isocitrate dehydrogenase 1 (NADP+), soluble |
| Hs.2064 | 529070 | AA064827 | vimentin |
| Hs.155079 | 41356 | R59165 | protein phosphatase 2, regulatory subunit B (B56), alpha isoform |
| Hs.77502 | 530811 | AA070029 | methionine adenosyltransferase II, alpha |
| Hs.275924 | 345643 | W71999 | dystrophia myotonica-containing WD repeat motif |
| Hs.227729 | 490760 | AA133163 | FK506-binding protein 2 (13kD) |
| Hs.52644 | 309290 | N93925 | SKAP55 homologue |
| Hs.115740 | 28572 | R40902 | KIAA0210 gene product |
| Hs.82285 | 28596 | R37481 | phosphoribosylglycinamide formyltransferase, phosphoribosylglycinamide synthetase, phosphoribosylaminoimidazole synthetase |
| Hs.75812 | 28884 | R40253 | phosphoenolpyruvate carboxykinase 2 (mitochondrial) |
| Hs.23978 | 363287 | AA019362 | scaffold attachment factor B |
| Hs.278857 | 45625 | H08426 | heterogeneous nuclear ribonucleoprotein H2 (H') |
| Hs.25035 | 666087 | AA193554 | chloride intracellular channel 4 like |
| Hs.234546 | 126487 | R06623 | GMPR2 for guanosine monophosphate reductase isolog |
| Hs.184760 | 264287 | N21190 | CCAAT-box-binding transcription factor |
| Hs.77929 | 128773 | R16755 | excision repair cross-complementing rodent repair deficiency, complementation group 3 (xeroderma pigmentosum group B complem |
| Hs.75932 | 34298 | R44350 | N-ethylmaleimide-sensitive factor attachment protein, alpha |
| Hs.157145 | 48300 | H14474 | tetracycline transporter-like protein |
| Hs.2227 | 612403 | AA179189 | CCAAT/enhancer binding protein (C/EBP), gamma |
| Hs.205842 | 220120 | H82605 | Homo sapiens mRNA; cDNA DKFZp434L231 (from clone DKFZp434L231) |
| Hs.99855 | 146605 | R79948 | formyl peptide receptor-like 1 |
| Hs.69423 | 526283 | AA079782 | kallikrein 10 |
| Hs.153678 | 485750 | AA039934 | reproduction 8 |
| Hs.279651 | 346688 | W74647 | melanoma inhibitory activity |
| Hs.169886 | 489919 | AA114835 | tenascin XA |
| Hs.1757 | 774100 | AA442123 | L1 cell adhesion molecule (hydrocephalus, stenosis of aqueduct of Sylvius 1, MASA (mental retardation, aphasia, shuffling ga |
| Hs.250911 | 545323 | AA076582 | interleukin 13 receptor, alpha 1 |
| Hs.25615 | 546600 | AA084517 | DnaJ-like heat shock protein 40 |
| Hs.76038 | 44975 | H08820 | isopentenyl-diphosphate delta isomerase |
| Hs.261285 | 550127 | AA1012 | pleiotropic regulator 1 (PRL1, Arabidopsis homolog) |
| Hs.931 | 562597 | AA086247 | myosin, heavy polypeptide 2, skeletal muscle, adult |
| Hs.108957 | 321973 | W37801 | 40S ribosomal protein S27 isoform |
| Hs.247309 | 123782 | R01201 | succinate-CoA ligase, GDP-forming, beta subunit |
| Hs.96247 | 645235 | AA199863 | translin-associated factor X |
| Hs.279946 | 32132 | R42928 | methionine-tRNA synthetase |
| Hs.75932 | 32531 | R43287 | N-ethylmaleimide-sensitive factor attachment protein, alpha |
| Hs.95998 | 126314 | R06415 | Friedreich ataxia |
| Hs.9908 | 73531 | T55560 | nitrogen fixation cluster-like |
| Hs.82043 | 33109 | R44799 | D123 gene product |
| Hs.67726 | 128000 | R09347 | macrophage receptor with collagenous structure |
| Hs.79187 | 265680 | N25352 | coxsackie virus and adenovirus receptor |
| Hs.748 | 47723 | H11614 | fibroblast growth factor receptor 1 (fins-related tyrosine kinase 2, Pfeiffer syndrome) |
| Hs.193852 | 199655 | R96618 | ATP-binding cassette, sub-family C (CFTR/MRP), member 2 |
| Hs.79748 | 267637 | N25433 | solute carrier family 3 (activators of dibasic and neutral amino acid transport), member 2 |
| Hs.154679 | 48114 | H11494 | synaptotagmin 1 |
| Hs.81915 | 382295 | AA062858 | leukemia-associated phosphoprotein p18 (stathmin) |
| Hs.73986 | 49237 | H15069 | CDC-like kinase 2 |
| Hs.194638 | 49548 | H15431 | polymerase (RNA) II (DNA directed) polypeptide D |
| Hs.78596 | 208005 | H60553 | proteasome (prosome, macropain) subunit, beta type, 5 |
| Hs.94498 | 277906 | N63398 | leukocyte immunoglobulin-like receptor, subfamily A (with TM domain), member 2 |
| Hs.167835 | 210862 | H65660 | acyl-Coenzyme A oxidase |
| Hs.149894 | 50754 | H18070 | mitochondrial translational initiation factor 2 |
| Hs.154437 | 22165 | T66157 | phosphodiesterase 2A, cGMP-stimulated |
| Hs.102876 | 328750 | W45402 | pancreatic lipase |
| Hs.199160 | 511356 | AA086055 | myeloid/lymphoid or mixed-lineage leukemia (trithorax (Drosophila) homolog) |
| Hs.91773 | 23534 | R38106 | protein phosphatase 2 (formerly 2A), catalytic subunit, alpha isoform |
| Hs.13370 | 24175 | R39324 | DKFZP564G0222 protein |
| Hs.75576 | 232629 | H72599 | plasminogen |
| Hs.80350 | 25263 | R12792 | protein phosphatase 2 (formerly 2A), catalytic subunit, beta isoform |
| Hs.83920 | 25457 | R39849 | peptidylglycine alpha-amidating monooxygenase |
| Hs.154510 | 529844 | AA070863 | carbonyl reductase 3 |
| Hs.78869 | 26531 | R38473 | transcription elongation factor A (SII), 1 |
| Hs.6762 | 26689 | R39132 | hypothetical protein |
| Hs.147176 | 531496 | AA074202 | Homo sapiens eps15R mRNA, partial cds |
| Hs.31472 | 27920 | R40486 | transforming growth factor beta-activated kinase-binding protein 1 |
| Hs.75551 | 43960 | H04825 | Ras suppressor protein 1 |
| Hs.198248 | 320285 | W04613 | UDP-Gal:betaGlcNAc beta 1,4-galactosyltransferase, polypeptide 1 |
| Hs.85937 | 561840 | AA086285 | myosin-binding protein C, fast-type |
| Hs.29222 | 125674 | R07481 | zinc finger protein 76 (expressed in testis) |
| Hs.597 | 32936 | R43808 | glutamic-oxaloacetictransaminase 1, soluble (aspartate aminotransferase 1) |
| Hs.75412 | 196501 | R91550 | Arginine-rich protein |
| Hs.239298 | 37210 | R50928 | microtubule-associated protein 4 |
| Hs.118725 | 51680 | H23998 | selenophosphate synthetase 2 |
| Hs.2043 | 526334 | AA079855 | adenine nucleotide translocator 1 (skeletal muscle) |
| Hs.142 | 526852 | AA113154 | sulfotransferase family 1A, phenol-preferring, member 1 |
| Hs.11223 | 28095 | R40321 | isocitrate dehydrogenase 1 (NADP+), soluble |
| Hs.194692 | 28615 | R40452 | cysteine desulfurase |
| Hs.108623 | 28330 | R40660 | thrombospondin 2 |
| Hs.110165 | 358675 | W94107 | ribosomal protein L26 homolog |
| Hs.165843 | 51528 | H20727 | casein kinase 2, beta polypeptide |
| Hs.108112 | 44319 | H05287 | histone fold protein CHRAC17; DNA polymerase epsilon p17 subunit |
| Hs.77917 | 29799 | R42127 | ubiquitin carboxyl-terminal esterase L3 (ubiquitin thiolesterase) |
| Hs.17958 | 120278 | T96983 | cerebroside (3'-phosphoadenylylsulfate:galactosylceramide 3') sulfotransferase |
| Hs.20912 | 179130 | H50183 | adenomatous polyposis coli like |
| Hs.279591 | 256357 | H94013 | Cyclin-dependent kinase 7 |
| Hs.36566 | 31097 | R41791 | LIM domain kinase 1 |
| Hs.78225 | 186308 | H29761 | annexin A1 |
| Hs.83484 | 68607 | T53300 | SRY (sex determining region Y)-box 4 |
| Hs.40735 | 364722 | AA024552 | frizzled (Drosophila) homolog 3 |
| Hs.5862 | 32316 | R42908 | hypothetical protein |
| Hs.251972 | 193602 | H47573 | complement component 3 |
| Hs.104433 | 665299 | AA195289 | Homo sapiens napsin 2 precursor, mRNA, partial sequence |
| Hs.113052 | 125148 | R05309 | RNA cyclase homolog |
| Hs.76494 | 565991 | AA121850 | proline arginine-rich end leucine-rich repeat protein |
| Hs.33713 | 203385 | H54815 | myo-inositol 1-phosphate synthase A1 |
| Hs.79709 | 49287 | H15647 | phosphotidylinositol transfer protein |
| Hs.11930 | 82941 | T69406 | nuclear receptor subfamily 0, group B, member 2 |
| Hs.75348 | 36060 | R46376 | proteasome (prosome, macropain) activator subunit 1 (PA28 alpha) |
| Hs.154320 | 273466 | N36891 | ubiquitin-activating enzyme E1C (homologous to yeast UBA3) |
| Hs.1189 | 208369 | H62837 | E2F transcription factor 3 |

**Table II**

| (Group II): | | | |
|---|---|---|---|
| Unigene Cluster | IMAGE ID | Genbank Accession no. | Putative gene name |
| Hs.107600 | 51686 | H24004 | EST |
| Hs.109212 | 190915 | H39221 | ESTs, Weakly similar to Kelch motif containing protein [H.sapiens] |
| NA | 80790 | T63042 | NA |
| Hs.30868 | 236388 | H62405 | ESTs |
| NA | 322334 | NA | NA |
| NA | 129855 | NA | NA |
| Hs.20588 | 210873 | H67736 | ESTs, Moderately similar to PAHO_HUMAN PANCREATIC HORMONE PRECURSOR [H.sapiens] |
| Hs.146215 | 153571 | R48535 | ESTs |
| Hs.222195 | 301238 | N80787 | ESTs |
| Hs.29403 | 156906 | R74233 | ESTs, Weakly similar to DDX8_HUMAN PROBABLE ATP-DEPENDENT RNA HELICASE HRH1 [H.sapiens] |
| Hs.168236 | 113949 | T79758 | ESTs |
| Hs.246023 | 264571 | N20320 | EST |
| NA | 129757 | NA | NA |
| NA | 108340 | T70599 | NA |
| Hs.117565 | 142760 | R71081 | ESTs |
| Hs.103420 | 471619 | AA035469 | ESTs |
| NA | 27300 | NA | NA |
| NA | 60298 | NA | NA |
| Hs.104623 | 214600 | H71226 | ESTs, Highly similar to KIAA0940 protein [H.sapiens] |
| NA | 53082 | NA | NA |
| Hs.74052 | 307138 | N93721 | ESTs |
| Hs.8977 | 67397 | T49325 | ESTs |
| NA | 567395 | AA130843 | NA |
| Hs.113980 | 202051 | R99560 | ESTs, Weakly similar to [Human endogenous retrovirus type C oncovirus sequence.], gene product [H.sapiens] |
| Hs.270197 | 66996 | T69707 | ESTs |
| NA | 213894 | H72939 | NA |
| Hs.119756 | 38816 | R49144 | ESTs |
| Hs.183071 | 108309 | T70568 | ESTs |
| Hs.36102 | 232772 | H72723 | ESTs, Highly similar to MT1B_HUMAN METALLOTHIONEIN-IB [H.sapiens] |
| NA | 67885 | T52774 | NA |
| Hs.203367 | 134322 | R31938 | ESTs |
| NA | 72672 | T50400 | NA |
| Hs.31171 | 148609 | H12612 | ESTs |
| NA | 530744 | AA069924 | NA |
| Hs.21667 | 245813 | N55301 | ESTs |
| Hs.12152 | 29602 | R42296 | ESTs, Moderately similar to SRPB_MOUSE SIGNAL RECOGNITION PARTICLE RECEPTOR BETA SUBUNIT [M.musculus] |
| Hs.93967 | 180161 | R85501 | ESTs |
| NA | 66469 | NA | NA |
| Hs.169161 | 35000 | R45094 | ESTs, Moderately similar to MAON_HUMAN NADP-DEPENDENT MALIC ENZYME, MITOCHONDRIAL PRECURSOR [H.sapiens] |
| Hs.18627 | 37178 | R49398 | ESTs, Weakly similar to GP36b glycoprotein [H.sapiens] |
| NA | 219689 | H80014 | NA |
| Hs.31848 | 525319 | AA069144 | ESTs, Weakly similar to hypothetical protein [H.sapiens] |
| NA | 526038 | AA076128 | NA |
| NA | 146842 | R80670 | NA |
| Hs.125042 | 239510 | H81265 | ESTs |
| NA | 545120 | AA075716 | NA |
| Hs.107382 | 44151 | H05814 | ESTs |
| NA | 29532 | R41566 | NA |
| Hs.125729 | 310600 | N99898 | ESTs, Weakly similar to zinc finger protein zfp31 [H.sapiens] |
| NA | 548932 | AA115168 | NA |
| NA | 124187 | R01274 | NA |
| Hs.31110 | 47703 | H12084 | ESTs, Weakly similar to MAGE-B4 [H.sapiens] |
| Hs.13155 | 67006 | T69711 | EST |
| NA | 530033 | AA070488 | NA |
| NA | 530736 | AA069921 | NA |
| NA | 531578 | AA074126 | NA |
| Hs.6820 | 28213 | R40787 | ESTs, Weakly similar to putative [C.elegans] |
| NA | 546973 | AA083382 | NA |
| Hs.91785 | 31123 | R42362 | ESTs |
| Hs.230064 | 108369 | T77828 | EST |
| Hs.265200 | 624513 | AA187228 | ESTs, Highly similar to S29331 glutamate dehydrogenase - human [H.sapiens] |
| Hs.22893 | 32643 | R43166 | ESTs |
| NA | 66347 | NA | NA |
| Hs.269425 | 381260 | AA057000 | ESTs, Highly similar to dJ283E3.6.1 [H.sapiens] |
| Hs.36269 | 416646 | W86446 | ESTs, Weakly similar to ODB2_HUMAN LIPOAMIDE ACYLTRANSFERASE COMPONENT OF BRANCHED-CHAIN ALPHA-KETO ACID DEHYDROGENASE COMPL |
| NA | 53024 | R15882 | NA |
| Hs.278871 | 51017 | H19309 | ESTs, Weakly similar to AC007228_2 BC37295_1 [H.sapiens] |
| Hs.10846 | 152989 | R50746 | ESTs, Weakly similar to JH0783 diamine N-acetyltransferase [H.sapiens] |
| Hs.137361 | 488337 | AA046643 | ESTs, Weakly similar to RAS-RELATED PROTEIN RAB-7 [H.sapiens] |
| NA | 530870 | AA070154 | NA |
| NA | 531411 | AA071566 | NA |
| NA | 544846 | AA075338 | NA |
| Hs.177276 | 202575 | H53817 | ESTs |
| Hs.191112 | 67022 | T69727 | ESTs |
| NA | 68185 | T52983 | NA |
| Hs.131897 | 67064 | T70341 | ESTs |
| Hs.24889 | 41388 | R56121 | ESTs |
| NA | 66327 | NA | NA |
| NA | 544820 | AA075280 | NA |
| Hs.143333 | 193846 | H51750 | EST |
| NA | 526156 | AA076627 | NA |
| NA | 530281 | AA112048 | NA |
| Hs.44426 | 346063 | W72646 | ESTs, Weakly similar to GSHH_HUMA1 PHOSPHOLIPID HYDROPEROXIDE GLUTATHIONE PEROXIDASE [H.sapiens] |
| NA | 545623 | AA078835 | NA |
| Hs.43897 | 257368 | N27163 | ESTs, Weakly similar to P2CA_HUMAN PROTEIN PHOSPHATASE 2C ALPHA ISOFORM [H.sapiens] |
| Hs.172080 | 129000 | R10363 | ESTs |
| Hs.146182 | 208169 | H60623 | ESTs, Weakly similar to lactase phlorizinhydrolase [H.sapiens] |
| Hs.251946 | 418006 | W90707 | ESTs, Moderately similar to PAB1_HUMAN POLYADENYLATE-BINDING PROTEIN 1 [H.sapiens] |
| Hs.177276 | 202575 | H53817 | ESTs |
| NA | 544954 | AA075353 | NA |
| Hs.269605 | 545077 | AA075680 | ESTs, Moderately similar to SUCCINAT DEHYDROGENASE [H.sapiens] |
| Hs.26481 | 43460 | H05933 | ESTs, Weakly similar to NS1-binding protein [H.sapiens] |
| Hs.9299 | 31987 | R43047 | ESTs |
| Hs.106356 | 32991 | R44770 | ESTs |
| Hs.173121 | 486296 | AA044079 | ESTs |
| NA | 545681 | AA079371 | NA |
| NA | 546318 | AA084033 | NA |
| Hs.261330 | 548702 | AA125823 | ESTs, Highly similar to dJ109F14.2 [H.sapiens] |
| NA | 69410 | NA | NA |
| NA | 67419 | T49342 | NA |
| Hs.134013 | 381968 | AA063646 | ESTs, Moderately similar to NK homeobox protein [H.sapiens] |

**Table III**

| (Group III): | | | |
|---|---|---|---|
| Unigene Cluster | IMAGE ID | Genbank Accession no. | Putative gene name |
| Hs.183864 | 328527 | W40254 | elastase 3B |
| NA | 119202 | T94099 | NA |
| Hs.30352 | 429312 | AA007373 | ribosomal protein S6 kinase, 52kD, polypeptide 1 |
| Hs.79474 | 38287 | R49224 | tyrosine 3-monooxygenase/tryptophan 5-monooxygenase activation protein, epsilon polypeptide |
| Hs.177592 | 141028 | R66697 | ribosomal protein, large, P1 |
| Hs.184093 | 510608 | AA099408 | HERV-H LTR-associating 1 |
| Hs.87149 | 290759 | N67642 | integrin, beta 3 (platelet glycoprotein IIIa, antigen CD61) |
| NA | 144825 | R76566 | NA |
| Hs.151123 | 290283 | N64471 | neuronal Shc |
| Hs.4217 | 145899 | R79161 | collagen, type VI, alpha 2 |
| Hs.100000 | 122381 | T99218 | S100 calcium-binding protein A8 (calgranulin A) |
| Hs.249982 | 40205 | R52103 | cathepsin B |
| Hs.4814 | 40617 | R55744 | mannosidase, alpha, class 1B, member 1 |
| Hs.154654 | 25594 | R15113 | cytochrome P450, subfamily I (dioxin-inducible), polypeptide 1 (glaucoma 3, primary infantile) |
| NA | 527085 | AA114048 | NA |
| Hs.2250 | 153025 | R50354 | leukemia inhibitory factor (cholinergic differentiation factor) |
| Hs.25590 | 153589 | R48580 | stanniocalcin |
| Hs.2633 | 108222 | T69781 | desmoglein 1 |
| Hs.85701 | 345430 | W72473 | phosphoinositide-3-kinase, catalytic, alpha polypeptide |
| Hs.211578 | 345935 | W72201 | MAD (mothers against decapentaplegic, Drosophila) homolog 3 |
| Hs.2030 | 205185 | H59861 | thrombomodulin |
| Hs.115396 | 155233 | R70379 | immunoglobulin heavy constant delta |
| Hs.366 | 489620 | AA099044 | 6-pyruvoyltetrahydropterin synthase |
| Hs.1675 | 309295 | N93935 | gamma-glutamyltransferase-like activity 1 |
| Hs.84229 | 38040 | R59398 | splicing factor, arginine/serine-rich 8 (suppressor-of-white-apricot, Drosophila homolog) |
| Hs.43857 | 113822 | T77062 | similar to glucosamine-6-sulfatases |
| Hs.55967 | 309974 | N99100 | short stature homeobox 2 |
| Hs.86347 | 31156 | R42595 | ESTs, Weakly similar to predicted using Genefinder [C.elegans] |
| Hs.79059 | 45133 | H07895 | transforming growth factor, beta receptor III (betaglycan, 300kD) |
| NA | 256612 | NA | NA |
| Hs.82269 | 258229 | N30652 | progestagen-associated endometrial protein (placental protein 14, pregnancy-associated endometrial alpha-2-globulin, alpha u |
| Hs.77840 | 261258 | H98114 | annexin A4 |
| Hs.9075 | 562928 | AA085850 | serine/threonine kinase 17a (apoptosis-inducing) |
| Hs.78056 | 32041 | R41770 | cathepsin L |
| Hs.789 | 323238 | W42723 | GRO1 oncogene (melanoma growth stimulating activity, alpha) |
| Hs.77572 | 47493 | H11583 | BCL2/adenovirus E1B 19kD-interacting protein 1 |
| Hs.107966 | 199180 | R95740 | paraoxonase 3 |
| Hs.22026 | 129610 | R16547 | ESTs |
| Hs.154443 | 200536 | R99175 | minichromosome maintenance deficient (S. cerevisiae) 4 |
| Hs.85137 | 591617 | AA158803 | cyclin A2 |
| Hs.31130 | 48276 | H12262 | transmembrane 7 superfamily member 2 |
| Hs.7645 | 201352 | R98600 | fibrinogen, B beta polypeptide |
| Hs.31137 | 66972 | T67544 | protein tyrosine phosphatase, receptor type, epsilon polypeptide |
| Hs.86347 | 270794 | N32932 | ESTs, Weakly similar to predicted using Genefinder [C.elegans] |
| Hs.83341 | 49318 | H15718 | AXL receptor tyrosine kinase |
| Hs.202362 | 270895 | N32504 | ESTs, Weakly similar to S71091 acetyl-CoA carboxylase [H.sapiens] |
| Hs.170114 | 270560 | N33237 | KIAA0061 protein |
| Hs.24309 | 133914 | R28671 | hypothetical protein FLJ11106 |
| NA | 108296 | T70555 | NA |
| Hs.94360 | 82205 | T68873 | metallothionein 1L |
| Hs.181392 | 135225 | R32850 | major histocompatibility complex, class I, E |
| Hs.81118 | 274197 | H49887 | leukotriene A4 hydrolase |
| Hs.104203 | 36992 | R48944 | ESTs |
| Hs.17411 | 327073 | W02696 | KIAA0699 protein |
| Hs.82269 | 327077 | W02698 | progestagen-associated endometrial protein (placental protein 14, pregnancy-associated endometrial alpha-2-globulin, alpha u |
| NA | 108418 | T77845 | NA |
| Hs.94382 | 279363 | N48691 | adenosine kinase |
| Hs.6456 | 211555 | H56330 | chaperonin containing TCP1, subunit 2 (beta) |
| Hs.169907 | 21994 | T66320 | glutathione S-transferase A4 |
| Hs.74561 | 428909 | AA004817 | alpha-2-macroglobulin |
| Hs.274260 | 108190 | T69749 | ATP-binding cassette, sub-family C (CFTR/MRP), member 6 |
| Hs.13225 | 22396 | T87624 | UDP-Gal:betaGlcNAc beta 1,4-galactosyltransferase, polypeptide 4 |
| Hs.79345 | 22304 | T82485 | coagulation factor VIIIc, procoagulant component (hemophilia A) |
| Hs.182490 | 286024 | N64275 | leucine-rich protein mRNA |
| Hs.75627 | 141979 | R69023 | CD14 antigen |
| Hs.153614 | 287575 | N62125 | retinitis pigmentosa GTPase regulator |
| Hs.20423 | 143388 | R74208 | NOT4 (negative regulator of transcription 4, yeast) homolog |
| NA | 52365 | H24082 | NA |
| Hs.194720 | 52150 | H24068 | ATP-binding cassette, sub-family G (WHITE), member 2 |
| Hs.75410 | 342231 | W61143 | heat shock 70kD protein 5 (glucose-regulated protein, 78kD) |
| Hs.799 | 24365 | R37964 | diphtheria toxin receptor (heparin-binding epidermal growth factor-like growth factor) |
| Hs.31819 | 52295 | H24360 | ESTs, Weakly similar to thioredoxin-like protein [H.sapiens] |
| Hs.3280 | 297727 | N69907 | caspase 6, apoptosis-related cysteine protease |
| Hs.18212 | 230060 | H68190 | DNA segment on chromosome X (unique) 9879 expressed sequence |
| Hs.77274 | 143356 | R74194 | plasminogen activator, urokinase |
| Hs.6066 | 24792 | R38781 | Rho guanine nucleotide exchange factor (GEF) 4 |
| Hs.228572 | 150162 | H04461 | EST |
| Hs.198891 | 40240 | R55052 | serine/threonine-protein kinase PRP4 homolog |
| NA | 231916 | H92881 | NA |
| Hs.118845 | 301128 | N81117 | troponin C, slow |
| Hs.75517 | 526215 | AA076664 | laminin, beta 3 (nicein (125kD), kalinin (140kD), BM600 (125kD)) |
| Hs.75819 | 40348 | R54793 | glycoprotein M6A |
| Hs.75354 | 40567 | R55251 | GCN1 (general control of amino-acid synthesis 1, yeast)-like 1 |
| Hs.63510 | 301301 | N80830 | KIAA0141 gene product |
| Hs.199533 | 21418 | T65374 | ESTs |
| NA | 238357 | H64393 | NA |
| Hs.7158 | 307072 | N89678 | DKFZP566H073 protein |
| Hs.167292 | 345648 | W72064 | ESTs, Weakly similar to zinc finger protein C2H2-25 [H.sapiens] |
| Hs.76366 | 345703 | W71991 | BCL2-antagonist of cell death |
| Hs.87149 | 200209 | R97831 | integrin, beta 3 (platelet glycoprotein IIIa, antigen CD61) |
| Hs.251653 | 41413 | R56886 | tubulin, beta, 2 |
| Hs.117852 | 238349 | H64389 | ATP-binding cassette, sub-family D (ALD), member 2 |
| Hs.115537 | 26189 | R20620 | ESTs, Weakly similar to |
| | | | MICROSOMAL DIPEPTIDASE PRECURSOR [H.sapiens] |
| Hs.7844 | 42075 | R60845 | golgi autoantigen, golgin subfamily b, macrogolgin (with transmembrane signal), 1 |
| Hs.1239 | 109164 | T81089 | alanyl (membrane) aminopeptidase (aminopeptidase N, aminopeptidase M, microsomal aminopeptidase, CD13, p150) |
| Hs.77899 | 307949 | N92266 | tropomyosin 1 (alpha) |
| Hs.8123 | 346824 | W78007 | chromobox homolog 3 (Drosophila HP1 gamma) |
| Hs.171731 | 240062 | H82236 | solute carrier family 14 (urea transporter), member 1 (Kidd blood group) |
| Hs.2551 | 241489 | H90431 | adrenergic, beta-2-, receptor, surface |
| Hs.184161 | 310131 | N98616 | exostoses (multiple) 1 |
| Hs.75596 | 376696 | AA046615 | interleukin 2 receptor, beta |
| Hs.1422 | 347751 | W81586 | Gardner-Rasheed feline sarcoma viral (v-fgr) oncogene homolog |
| Hs.507 | 357785 | W95595 | comeodesmosin |
| Hs.75445 | 27542 | R40157 | SPARC-like 1 (mast9, hevin) |
| Hs.26691 | 43006 | R59736 | ESTs |
| NA | 163482 | H14182 | NA |
| NA | 544875 | AA075381 | NA |
| Hs.168236 | 113951 | T79759 | ESTs |
| Hs.256309 | 309944 | N94512 | Human beta-1D integrin mRNA, cytoplasmic domain, partial cds |
| Hs.182741 | 310447 | N98462 | T1A1 cytotoxic granule-associated RNA-binding protein-like 1 |
| Hs.215595 | 120148 | T95078 | guanine nucleotide binding protein (G protein), beta polypeptide 1 |
| NA | 547027 | AA082916 | NA |
| NA | 68351 | NA | NA |
| Hs.8015 | 44909 | H07857 | ubiquitin specific protease 21 |
| Hs.9475 | 67188 | T52634 | ESTs, Weakly similar to GTRS_HUMAN GLUCOSE TRANSPORTER TYPE 5, SMALL INTESTINE [H.sapiens] |
| Hs.234773 | 509919 | AA056421 | ecotropic viral integration site 1 |
| Hs.34853 | 260740 | H97932 | inhibitor of DNA binding 4, dominant negative helix-loop-helix protein |
| Hs.155606 | 364554 | AA022577 | paired mesoderm homeo box 1 |
| Hs.154095 | 261759 | H99156 | zinc finger protein 143 (clone pHZ-1) |
| NA | 322339 | NA | NA |
| Hs.81737 | 262750 | H99622 | palmitoyl-protein thioesterase 2 |
| Hs.181125 | 194467 | R83196 | immunoglobulin lambda locus |
| Hs.278607 | 263725 | H99680 | ubiquitin activating enzyme E1-like |
| | | | protein |
| Hs.404 | 46936 | H10052 | myeloid/lymphoid or mixed-lineage |
| | | | leukemia (trithorax (Drosophila) homolog); translocated to, 3 |
| Hs.1197 | 128225 | R11507 | heat shock 10kD protein 1 (chaperonin 10) |
| Hs.83050 | 128937 | R10699 | phosphoinositide-3-kinase, regulatory subunit 4, p150 |
| Hs.1012 | 129270 | R11065 | complement component 4-binding protein, alpha |
| Hs.274260 | 200946 | R97755 | ATP-binding cassette, sub-family C (CFTR/MRP), member 6 |
| Hs.28532 | 68011 | T49766 | ESTs, Weakly similar to BAI1-associated protein 1 [H.sapiens] |
| Hs.76722 | 594019 | AA165157 | CCAAT/enhancer binding protein (C/EBP), delta |
| Hs.75621 | 78425 | T61377 | protease inhibitor 1 (anti-elastase), alpha-1-antitrypsin |
| Hs.9994 | 201995 | R99339 | lipase, hepatic |
| Hs.74111 | 49402 | H15565 | RNA-binding protein (autoantigenic) |
| Hs.848 | 610317 | AA171524 | FK506-binding protein 4 (59kD) |
| Hs.73853 | 612944 | AA181547 | bone morphogenetic protein 2 |
| Hs.76688 | 83060 | T67816 | carboxylesterase 1 (monocyte/macrophage serine esterase 1) |
| Hs.197728 | 85578 | T72257 | carboxylesterase 2 (intestine, liver) |
| Hs.75155 | 85640 | T62051 | transferrin |
| Hs.78036 | 137257 | R36683 | solute carrier family 6 (neurotransmitter transporter, noradrenalin), member 2 |
| Hs.107082 | 139304 | R63714 | ESTs, Moderately similar to alternatively spliced product using exon 13A [H.sapiens] |
| Hs.6518 | 50866 | H17125 | ganglioside expression factor 2 |
| Hs.198253 | 139530 | R62322 | major histocompatibility complex, class II, DQ alpha 1 |
| Hs.265262 | 141115 | R66326 | colony stimulating factor 2 receptor, beta, low-affinity (granulocyte-macrophage) |
| Hs.124186 | 141298 | R63802 | ring finger protein 2 |
| Hs.82985 | 340928 | W57799 | collagen, type V, alpha 2 |
| Hs.155924 | 23235 | R39184 | cAMP responsive element modulator |
| Hs.272630 | 512924 | AA063307 | vacuolar proton pump delta polypeptide |
| Hs.155140 | 21658 | T65122 | casein kinase 2, alpha 1 polypeptide |
| NA | 145407 | R78034 | NA |
| Hs.182490 | 53071 | R16051 | leucine-rich protein mRNA |
| Hs.184669 | 153055 | R50369 | zinc finger protein 144 (Mel-18) |
| Hs.50651 | 171569 | H18190 | Janus kinase 1 (a protein tyrosine kinase) |
| Hs.83393 | 344997 | W72895 | cystatin E/M |
| Hs.268915 | 108235 | T69792 | ESTs |
| Hs.263671 | 488635 | AA044896 | Homo sapiens mRNA; cDNA |
| | | | DKFZp434I0812 (from clone DKFZp434I0812); partial cds |
| Hs.85701 | 250142 | N23534 | phosphoinositide-3-kinase, catalytic, alpha polypeptide |
| Hs.67397 | 530888 | AA069960 | homeo box A1 |
| Hs.153612 | 308682 | N95462 | ATP-binding cassette, sub-family F (GCN20), member 2 |
| Hs.9873 | 244132 | N52439 | Homo sapiens mRNA; cDNA DKFZp434E0620 (from clone DKFZp434E0620); partial cds |
| NA | 544792 | AA075319 | NA |
| Hs.75682 | 43714 | H05738 | autoantigen |
| Hs.251972 | 28386 | R37386 | complement component 3 |
| Hs.155392 | 43852 | H04819 | collapsin response mediator protein 1 |
| NA | 545884 | AA079529 | NA |
| Hs.98493 | 29438 | R41276 | X-ray repair complementing defective repair in Chinese hamster cells 1 |
| Hs.64016 | 250640 | H98523 | protein S (alpha) |
| Hs.119251 | 546466 | AA084355 | ubiquinol-cytochrome c reductase core protein I |
| Hs.77448 | 44289 | H06253 | aldehyde dehydrogenase 4 (glutamate gamma-semialdehyde dehydrogenase; pyrroline-5-carboxylate dehydrogenase) |
| Hs.1139 | 360293 | AA013183 | cold shock domain protein A |
| Hs.41066 | 252534 | H87476 | ESTs, Moderately similar to EFGM_RAT ELONGATION FACTOR G, MITOCHONDRIAL PRECURSOR [R.norvegicus] |
| NA | 547154 | AA084870 | NA |
| Hs.18894 | 320170 | W04536 | adaptor-related protein complex 1, mu 2 subunit |
| Hs.160318 | 561873 | AA086437 | FXYD domain-containing ion transport regulator 1 (phospholemman) |
| Hs.234773 | 625011 | AA181023 | ecotropic viral integration site 1 |
| Hs.151573 | 33049 | R44018 | cryptochrome 1 (photolyase-like) |
| Hs.179606 | 46631 | H10061 | nuclear RNA helicase, DECD variant of DEAD box family |
| Hs.30965 | 46843 | H10072 | neuronal Shc adaptor homolog |
| Hs.107444 | 46667 | H09790 | Homo sapiens cDNA FLJ20562 fis, clone KAT11992 |
| Hs.154103 | 127614 | R09181 | LIM protein (similar to rat protein kinase C-binding enigma) |
| NA | 75009 | T51849 | NA |
| Hs.25615 | 200648 | R99249 | CGI-119 protein |
| Hs.78225 | 267361 | N24938 | annexin A1 |
| Hs.118666 | 49272 | H16503 | Human clone 23759 mRNA, partial cds |
| Hs.166017 | 268834 | N26000 | microphthalmia-associated transcription factor |
| Hs.77424 | 81221 | T57079 | Fc fragment of IgG, high affinity Ia, receptor for (CD64) |
| NA | 611899 | AA178923 | NA |
| Hs.160786 | 83166 | T68162 | argininosuccinate synthetase |
| Hs.19121 | 36905 | R49169 | adaptor-related protein complex 2, alpha 2 subunit |
| Hs.89649 | 49995 | H28958 | epoxide hydrolase 1, microsomal (xenobiotic) |
| Hs.84981 | 36341 | R62442 | X-ray repair complementing defective repair in Chinese hamster cells 5 (double-strand-break rejoining; Ku autoantigen, 80kD) |
| Hs.241567 | 139988 | R64674 | RNA binding motif, single stranded interacting protein 1 |
| Hs.78353 | 21899 | T65211 | SFRS protein kinase 2 |
| Hs.75613 | 429981 | AA034145 | CD36 antigen (collagen type I receptor, thrombospondin receptor) |
| Hs.4096 | 340876 | W57561 | KIAA0742 protein |
| Hs.75428 | 48198 | H11120 | superoxide dismutase 1, soluble (amyotrophic lateral sclerosis 1 (adult)) |
| NA | 513140 | AA063384 | NA |
| Hs.239176 | 148379 | H13300 | insulin-like growth factor 1 receptor |
| NA | 235880 | H52231 | NA |
| Hs.203246 | 239973 | H79874 | ESTs, Moderately similar to ZN91_HUMAN ZINC FINGER PROTEIN 91 [H.sapiens] |
| Hs.75511 | 267256 | N23360 | connective tissue growth factor |
| Hs.180383 | 42464 | R59865 | dual specificity phosphatase 6 |
| Hs.15114 | 489708 | AA099583 | ras homolog gene family, member D |
| NA | 530923 | AA070369 | NA |
| Hs.155020 | 27326 | R37020 | putative methyltransferase |
| Hs.131255 | 245269 | N54563 | ubiquinol-cytochrome c reductase binding protein |
| Hs.1252 | 356915 | W92730 | apolipoprotein H (beta-2-glycoprotein I) |
| Hs.2430 | 501972 | AA128103 | transcription factor-like 1 |
| Hs.21618 | 28938 | R40823 | ESTs |
| Hs.76392 | 309912 | N94493 | aldehyde dehydrogenase 1, soluble |
| Hs.1521 | 503206 | AA148925 | immunoglobulin mu binding protein 2 |
| Hs.2780 | 155061 | R70216 | jun D proto-oncogene |
| NA | 546664 | AA084411 | NA |
| Hs.169921 | 548957 | AA115186 | general transcription factor II, i, pseudogene 1 |
| Hs.151236 | 547732 | AA084099 | highly charged protein |
| Hs.173135 | 67286 | T49194 | dual-specificity tyrosine-(Y)-phosphorylation regulated kinase 2 |
| Hs.153998 | 363167 | AA019082 | creatine kinase, mitochondrial 1 (ubiquitous) |
| Hs.155597 | 257625 | N30864 | D component of complement (adipsin) |
| Hs.75111 | 45354 | H09725 | protease, serine, 11 (IGF binding) |
| Hs.70266 | 62112 | T41077 | yeast Sec31p homolog |
| Hs.217493 | 624382 | AA182794 | annexin A2 |
| Hs.197289 | 187804 | H44007 | rab3 GTPase-activating protein, non-catalytic subunit (150kD) |
| Hs.106674 | 46154 | H09066 | BRCA1 associated protein-1 (ubiquitin carboxy-terminal hydrolase) |
| Hs.77805 | 32649 | R43304 | ATPase, H+ transporting, lysosomal (vacuolar proton pump) 31kD; Vacuolar proton-ATPase, subunit E; V-ATPase, subunit E |
| Hs.123122 | 667355 | AA228030 | FSH primary response (LRPR1, rat) homolog 1 |
| Hs.66731 | 667188 | AA236353 | homeo box B13 |
| Hs.13776 | 127047 | R07880 | ADP-ribosyltransferase 4 |
| NA | 66315 | NA | NA |
| NA | 66492 | NA | NA |
| Hs.117077 | 586811 | AA130717 | zinc finger protein 264 |
| Hs.267887 | 33005 | R44793 | adenylyl cyclase-associated protein 2 |
| Hs.184771 | 265874 | N20996 | nuclear factor I/C (CCAAT-binding transcription factor) |
| Hs.12653 | 66810 | T64945 | ESTs |
| NA | 66814 | T64947 | NA |
| Hs.12107 | 267725 | N25578 | putative breast adenocarcinoma marker (32kD) |
| Hs.250666 | 130900 | R22228 | hairy (Drosophila)-homolog |
| Hs.183551 | 66986 | T69703 | EST |
| Hs.8110 | 593119 | AA160915 | adducin 3 (gamma) |
| Hs.10684 | 48653 | H14599 | Homo sapiens clone 24421 mRNA sequence |
| Hs.13880 | 35391 | R45220 | CGI-143 protein |
| Hs.82911 | 49507 | H15572 | protein tyrosine phosphatase type IVA, member 2 |
| Hs.7912 | 35271 | R45583 | neuronal cell adhesion molecule |
| Hs.5636 | 35530 | R45336 | RAB6, member RAS oncogene family |
| Hs.23016 | 35630 | R45296 | Human orphan G protein-coupled receptor (RDC1) mRNA, partial cds |
| Hs.142258 | 613363 | AA180403 | signal transducer and activator of transcription 3 (acute-phase response factor) |
| Hs.41639 | 273845 | N37094 | programmed cell death 2 |
| Hs.74583 | 50055 | H17451 | KIAA0275 gene product |
| Hs.271363 | 108322 | T70583 | ESTs |
| Hs.174185 | 108458 | T80109 | phosphodiesterase I/nucleotide pyrophosphatase 2 (autotaxin) |
| Hs.280666 | 37049 | R49483 | Homo sapiens chromosome 19, cosmid R32184 |
| NA | 108333 | T70597 | NA |
| Hs.192245 | 108461 | T80112 | ESTs |

**Table 4.**

| Group 4 Genes | | | |
|---|---|---|---|
| Unigene Cluster | IMAGE ID | Genbank Accession no. | Putative gene name |
| Hs.172028 | 212359 | H69859 | a disintegrin and metalloprotease domain 10 |
| Hs.173310 | 49387 | H15537 | protocadherin gamma subfamily C, 3 |
| Hs.83169 | 325050 | W49497 | matrix metalloproteinase 1 (interstitial collagenase) |
| Hs.34073 | 155834 | R86708 | BH-protocadherin (brain-heart) |
| Hs.118638 | 323236 | W42722 | non-metastatic cells 1, protein (NM23A) expressed in |
| Hs.154057 | 154770 | R55625 | matrix metalloproteinase 19 |
| Hs.275243 | 526111 | AA076242 | S100 calcium-binding protein A6 (calcyclin) |
| Hs.275163 | 325115 | W47002 | non-metastatic cells 2, protein (NM23B) expressed in |
| Hs.58324 | 345484 | W72552 | a disintegrin-like and metalloprotease (reprolysin type) with thrombospondin type 1 motif, 5 (aggrecanase-2) |
| Hs.118512 | 469969 | AA029934 | integrin, alpha V (vitronectin receptor, alpha polypeptide, antigen CD51) |
| Hs.2442 | 529120 | AA065135 | a disintegrin and metalloproteinase domain 9 (meltrin gamma) |
| Hs.2399 | 270505 | N33214 | matrix metalloproteinase 14 (membrane-inserted) |
| Hs.118638 | 81478 | T63504 | non-metastatic cells 1, protein (NM23A) expressed in |
| Hs.275243 | 326169 | W52162 | S100 calcium-binding protein A6 (calcyclin) |
| Hs.155392 | 43852 | H04819 | collapsin response mediator protein 1 |

### DETAILED DESCRIPTION

### Metastasis-Associated Genes

A model system containing model cell lines has been developed from a human lung adenocarcinoma cell line. The model cell lines, such as CL₁₋₀ and its sublines (e.g., CL₁₋₁ and CL₁₋₅), which are clonally related, have different invasion capabilities both *in vitro* and *in vivo.* To define genetic determinants of tumor metastasis, a cDNA microarray containing 9600 putative genes has been used to compare gene expression between the clonally related high metastatic and low metastatic tumors. Hundreds of genes have been identified that are differentially expressed in those model cell lines. Some of these genes, i.e., Group I, show strong correlation, either positively or negatively, between their expression levels and the invasiveness of cell lines. These findings illustrate that those model cell lines with varying invasive capabilities, together with a cDNA microarray technique, can be a good model system in identifying invasion or metastasis-associated genes.

Self-organizing maps (SOMs, see, Tamayo *et al*. (1999) *Proc. Natl. Acad. Sci. USA* 96: 2907), one of the widely used clustering methods, can organize expression profiles into clusters of patterns. This characteristic is useful to identify metastasis-associated genes. By using SOMs, 8,525 genes were analyzed and their expression profiles grouped into 100 clusters. Four of the clusters contained genes whose expression correlated positively with invasiveness of tumor cell lines; while another four clusters had negative correlation to invasiveness. Thus identified genes can be further confirmed by using Northern blotting and flow cytometric analysis. These genes sequences can be verified by re-sequencing.

The cDNA array has identified three groups of genes as being associated with tumor metastasis. Group I genes have known cellular functions that include, but are not limited to, proteases and adhesion molecules, cell cycle regulators, signal transduction molecules, cytoskeleton and motility proteins, urokinase-type plasminogen activators, and angiogenesis-related molecules. Microarray analysis also suggests that high expression level of tumor-associated antigen L6 is closely correlated with tumor metastasis. Group II genes are anonymous and strongly correlated either positively or negatively with invasiveness. Group III genes have known or unknown cellular functions and moderately correlated either positively or negatively with invasiveness.

### Arrays

Arrays are useful molecular tools for characterizing a sample by multiple criteria. For example, an array having a capture probes for one or more nucleic acids of Group I, II, III, or IV can be used to assess a metastasis state of cell. Arrays can have many addresses on a substrate. The featured arrays can be configured in a variety of formats, non-limiting examples of which are described below.

A substrate can be opaque, translucent, or transparent. The addresses can be distributed, on the substrate in one dimension, e.g., a linear array; in two dimensions, e.g., a planar array; or in three dimensions, e.g., a three dimensional array. The solid substrate may be of any convenient shape or form, e.g., square, rectangular, ovoid, or circular. Non-limiting examples of two-dimensional array substrates include glass slides, quartz (e.g., UV-transparent quartz glass), single crystal silicon, wafers (e.g., silica or plastic), mass spectroscopy plates, metal-coated substrates (e.g., gold), membranes (e.g., nylon and nitrocellulose), plastics and polymers (e.g., polystyrene, polypropylene, polyvinylidene difluoride, poly-tetrafluoroethylene, polycarbonate, PDMS, nylon, acrylic, and the like). Three-dimensional array substrates include porous matrices, e.g., gels or matrices. Potentially useful porous substrates include: agarose gels, acrylamide gels, sintered glass, dextran, meshed polymers (e.g., macroporous crosslinked dextran, sephacryl, and sepharose), and so forth.

An array can have a density of at least than 10, 50, 100, 200, 500, 1 000, 2 000, 10⁴, 10⁵, 10⁶, 10⁷, 10⁸, or 10⁹ or more addresses per cm² and ranges between. In some embodiments, the plurality of addresses includes at least 10, 100, 500, 1 000, 5 000, 10 000, or 50 000 addresses. In some other embodiments, the plurality of addresses includes less than 9, 99, 499, 999, 4 999, 9 999, or 49 999 addresses. Addresses in addition to the address of the plurality can be disposed on the array. The center to center distance can be 5 mm, 1 mm, 100 um, 10 um, 1um or less. The longest diameter of each address can be 5 mm, 1 mm, 100 um, 10 um, 1um or less. Each addresses can contain 0.1 ug, 1 ug, 100 ng, 10 ng, 1 ng, 100 pg, 10 pg, 1 pg, 0.1 pg or less of a capture agent, i.e. the capture probe. For example, each address can contain 100, 10³, 10⁴, 10⁵, 10⁶, 10⁷, 10⁸, or 10⁹ or more molecules of the nucleic acid.

A nucleic array can be fabricated by a variety of methods, e.g., photolithographic methods (see, e.g., U.S. Patent Nos. 5,143,854; 5,510,270; and. 5,527,681), mechanical methods (e.g., directed-flow methods as described in U.S. Patent No. 5,384,261), pin based methods (e.g., as described in U.S. Pat. No. 5,288,514), and bead based techniques (e.g., as described in PCT US/93/04145). A capture probe can be a single-stranded nucleic acid, a double-stranded nucleic acid (e.g., which is denatured prior to or during hybridization), or a nucleic acid having a single-stranded region and a double-stranded region. The capture probe can be selected by a variety of criteria, and can be designed by a computer program with optimization parameters. The capture probe can be selected to hybridize to a sequence rich (e.g., non-homopolymeric) region of a nucleic acid. The Tₘ of the capture probe can be optimized by prudent selection of the complementarity region and length. Ideally, the Tₘ of all capture probes on the array is similar, e.g., within 20, 10, 5, 3, or 2°C of one another. A database scan of available sequence information for a species can be used to determine potential cross-hybridization and specificity problems.

A nucleic acid array can be used to hybridize a nucleic acid that is obtained as follows: A RNA can be isolated by routine methods, e.g., including DNase treatment to remove genomic DNA and hybridization to an oligo-dT coupled a solid substrate (e.g., as described in *Current Protocols in Molecular Biology*, John Wiley & Sons, N.Y). The solid substrate is washed and the RNA is eluted. The RNA can be reversed transcribed and, optionally, amplified. The amplified nucleic acid can be labeled during amplification, e.g., by the incorporation of a labeled nucleotide. Examples of labels include fluorescent labels, e.g., red-fluorescent dye Cy5 (Amersham) or green-fluorescent dye Cy3 (Amersham), chemiluminescent labels, e.g., as described in U.S. Patent No. 4,277,437, and colorimetric detection, as described in Examples. Alternatively, the amplified nucleic acid can be labeled with biotin and detected after hybridization with labeled streptavidin, e.g., streptavidin-phycoerythrin (Molecular Probes). The labeled nucleic acid can be contacted to the array. In addition, a control nucleic acid or a reference nucleic acid can be contacted to the same array. The control nucleic acid or reference nucleic acid can be labeled with a label other than the sample nucleic acid, e.g., one with a different emission maximum. Labeled nucleic acids can be contacted to an array under hybridization conditions. The array can be washed, and then imaged to detect, e.g., color development or fluorescence, at each address of the array.

A polypeptide array can be used to determine the expression level of a polypeptide encoded by a nucleic acid selected from Group I, II, III, or IV. The polypeptide array can have antibody capture probes for each of the polypeptides.

A low-density (96 well format) polypeptide array has been developed in which polypeptides are spotted onto a nitrocellulose membrane (e.g., Ge, H. (2000) *Nucleic Acids Res.* 28: e3, I-VII). A high-density polypeptide array (100,000 samples within 222 x 222 mm) used for antibody screening was formed by spotting proteins onto polyvinylidene difluoride (PVDF) (e.g., Lueking *et al.* (1999) *Anal. Biochem.* 270: 103-111). Polypeptides can be printed on a flat glass plate that contained wells formed by an enclosing hydrophobic Teflon mask (e.g., Mendoza, *et al.* (1999). *Biotechniques* 27: 778-788.). Also, polypeptides can be covalently linked to chemically derivatized flat glass slides in a high-density array (1600 spots per square centimeter) (MacBeath, G, and Schreiber, S.L. (2000) *Science* 289: 1760-1763). De Wildt *et al.,* describe a high-density array of 18,342 bacterial clones, each expressing a different single-chain antibody, in order to screen antibody-antigen interactions (De Wildt *et al.* (2000). *Nature Biotech.* 18: 989-994). These art-known methods and others can be used to generate an array of antibodies for detecting the abundance of polypeptides in a sample. The sample can be labeled, e.g., biotinylated, for subsequent detection with streptavidin coupled to a fluorescent label. The array can then be scanned to measure binding at each address.

The nucleic acid and polypeptide arrays of the invention can be used in wide variety of applications. For example, the arrays can be used to analyze a patient sample. The sample is compared to data obtained previously, e.g., known clinical specimens or other patient samples. Further, the arrays can be used to characterize a cell culture sample, e.g., to determine a cellular state after varying a parameter, e.g., exposing the cell culture to an antigen, a transgene, or a test compound.

### Evaluating Expression

The level of expression of at least one nucleic acid selected from Group I, II, III, or IV can be measured in a number of ways, including, but not limited to: measuring the abundance of an mRNA encoded by a nucleic acid selected from Group I, II, III, or IV; measuring the amount of a polypeptide encoded by such a nucleic acid; or measuring an activity of a polypeptide encoded by such a nucleic acid.

The level of mRNA corresponding to a nucleic acid selected from Group I, II, III, or IV in a cell can be determined by the following formats. The isolated mRNA can be used in hybridization or amplification assays that include, but are not limited to, Northern analyses, polymerase chain reaction analyses, and probe arrays. One method for the detection of mRNA levels involves contacting the isolated mRNA with a nucleic acid probe that can hybridize to the mRNA encoded by the nucleic acid being detected. The nucleic acid probe can be, for example, a full-length of a nucleic acid complementary to a nucleic acid selected from Group I, II, III, or IV, or a portion thereof, such as an oligonucleotide of at least 7, 15, 30, 50, 100 nucleotides in length and sufficient to specifically hybridize under stringent conditions to the mRNA. In one format, mRNA is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA is contacted with the probes, for example, in a two-dimensional array.

The level of mRNA in a sample that is encoded by a nucleic acid selected from Group I, II, III, or IV can be evaluated using nucleic acid amplification, e.g., by rtPCR (Mullis (1987) U.S. Patent No. 4,683,202), ligase chain reaction (Barany (1991) *Proc. Natl. Acad. Sci. USA* 88:189-193), self sustained sequence replication (Guatelli *et al.* (1990) *Proc. Natl. Acad. Sci. USA* 87: 1874-1878), transcriptional amplification system (Kwoh *et al.* (1989), *Proc. Natl. Acad. Sci. USA* 86: 1173-1177), Q-Beta Replicase (Lizardi *et al.* (1988) *Bio*/*Technology* 6: 1197), rolling circle replication (Lizardi *et al.,* U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers. For an *in situ* format, a cell or tissue sample can be prepared/processed and immobilized on a support, typically a glass slide, and then contacted with a probe that can hybridize to mRNA that encodes the nucleic acid being analyzed.

A variety of methods can be used to determine the abundance of a polypeptide encoded by a nucleic acid selected from Group I, II, III, or IV. In general, these methods include contacting an agent that selectively binds to the polypeptide, such as an antibody, with a sample, to evaluate the level of the polypeptide in the sample. In some embodiments, the antibody bears a detectable label or is recognizable by a labeling agent. Antibodies can be polyclonal, or more preferably, monoclonal. An intact antibody, or a fragment thereof (e.g., Fab or F(ab')₂) can be used. The term "labeled," with regard to the probe or antibody, is intended to encompass direct labeling of the probe or the antibody by coupling (i.e., physically linking) a detectable substance to the probe or the antibody, as well as indirect labeling of the probe or antibody by reactivity with a detectable substance. Examples of detectable substances are provided herein.

The detection methods can be used to detect a polypeptide in a sample *in vitro* as well as *in vivo*. *In vitro* techniques for detection of the protein include enzyme linked immunosorbent assays (ELISAs), immunoprecipitations, immunofluorescence, enzyme immunoassay (EIA), radioimmunoassay (RIA), and Western blot analysis. *In vivo* techniques for detection the protein include introducing into a subject a labeled antibody. For example, the antibody can be labeled with a radioactive marker whose presence and location in a subject can be detected by standard imaging techniques. In another example, the sample can be labeled, e.g., biotinylated and then contacted to the antibody, e.g., an antibody positioned on an antibody array (as described below). The sample can be detected, e.g., with avidin coupled to a fluorescent label.

### Expression Profiles

A general scheme for producing and evaluating profiles is as follows. Nucleic acid is prepared from a sample, e.g., a sample of interest and hybridized to an array, e.g., with multiple addresses. Hybridization of the nucleic acid to the array is detected. The extent of hybridization at an address is represented by a numerical value and stored, e.g., in a vector, a one-dimensional matrix, or one-dimensional array. The vector **x** {Xₐ, x_{b} ...} has a value for each address of the array. For example, a numerical value for the extent of hybridization at a first address is stored in the variable xₐ. The numerical value can be adjusted, e.g., for local background levels, sample amount, and other variations. Nucleic acid is also prepared from a reference sample and hybridized to an array (e.g., the same or a different array), e.g., with multiple addresses. The vector y is construct identically to vector x. The sample expression profile and the reference profile can be compared, e.g., using a mathematical equation that is a function of the two vectors. The comparison can be evaluated as a scalar value, e.g., a score representing similarity of the two profiles. Either or both vectors can be transformed by a matrix in order to add weighting values to different nucleic acids detected by the array.

The expression data can be stored in a database, e.g., a relational database such as a SQL database (e.g., Oracle or Sybase database environments). The database can have multiple tables. For example, raw expression data can be stored in one table, wherein each column corresponds to a nucleic acid being assayed, e.g., an address or an array, and each row corresponds to a sample. A separate table can store identifiers and sample information, e.g., the batch number of the array used, date, and other quality control information.

Nucleic acids that are similarly regulated can be identified by clustering expression data to identify coregulated nucleic acids. Nucleic acids can be clustered using hierarchical clustering (see, e.g., Sokal and Michener (1958) *Univ. Kans. Sci. Bull.* 38: 1409), Bayesian clustering, k-means clustering, and self-organizing maps (see, Tamayo *et al.* (1999) *Proc. Natl. Acad. Sci. USA* 96: 2907).

Expression profiles obtained from nucleic acid expression analysis on an array can be used to compare samples and/or cells in a variety of states as described in Golub *et al.* ((1999) *Science* 286: 531). For example, multiple expression profiles from different conditions and including replicates or like samples from similar conditions are compared to identify nucleic acids whose expression level is predictive of the sample and/or condition. Each candidate nucleic acid can be given a weighted "voting" factor dependent on the degree of correlation of the nucleic acid's expression and the sample identity. A correlation can be measured using a Euclidean distance or a correlation coefficient, e.g., the Pearson correlation coefficient.

The similarity of a sample expression profile to a predictor expression profile (e.g., a reference expression profile that has associated weighting factors for each nucleic acid) can then be determined, e.g., by comparing the log of the expression level of the sample to the log of the predictor or reference expression value and adjusting the comparison by the weighting factor for all nucleic acids of predictive value in the profile.

### Transactional Methods for Evaluating a Sample

A transactional method for evaluating a sample can be performed as follows. A patient is treated by a physician. The physician obtains a sample (i.e., "patient sample"), e.g., a blood sample, from the patient. The patient sample can be delivered to a diagnostics department which can collate information about the patient, the patient sample, and results of the evaluation. A courier service can deliver the sample to a diagnostic service. Location of the sample is monitored by a courier computer system, and can be tracked by accessing the courier computer system, e.g., using a web page across the Internet. At the diagnostic service, the sample is processed to produce a sample expression profile. For example, nucleic acid is extracted from the sample, optionally amplified, and contacted to a nucleic acid microarray. Binding of the nucleic acid to the microarray is quantitated by a detector that streams data to the array diagnostic server. The array diagnostic server processes the microarray data, e.g., to correct for background, sample loading, and microarray quality. It can also compare the raw or processed data to a reference expression profile, e.g., to produce a difference profile. The raw profiles, processed profiles and/or difference profiles are stored in a database server. A network server manages the results and information flow. In one embodiment, the network server encrypts and compresses the results for electronic delivery to the healthcare provider's internal network. The results can be sent across a computer network, e.g., the Internet, or a proprietary connection. For data security, the diagnostic systems and the healthcare provider systems can be located behind firewalls. In another embodiment, an indication that the results are available can also be sent to the healthcare provider and/or the patient, for example, by to an email client. The healthcare provider, e.g., the physician, can access the results, e.g., using the secure Hypertext Transfer Protocol (HTTP) (e.g., with secure sockets layer (SSL) encryption). The results can be provided by the network server as a web page (e.g., in HTML, XML, and the like) for viewing on the physician's browser.

Further communication between the physician and the diagnostic service can result in additional tests, e.g., a second expression profile can be obtained for the sample, e.g., using the same or a different microarray.

### Screening a Test Compound

The invention provides a method for screening a test compound useful in the prevention or treatment of tumor metastasis. A "test compound" can be any chemical compound, for example, a macromolecule (e.g., a polypeptide, a protein complex, or a nucleic acid) or a small molecule (e.g., an amino acid, a nucleotide, an organic or inorganic compound). The test compound can have a formula weight of less than about 10,000 grams per mole, less than 5,000 grams per mole, less than 1,000 grams per mole, or less than about 500 grams per mole. The test compound can be naturally occurring (e.g., a herb or a nature product), synthetic, or both. Examples of macromolecules are proteins, protein complexes, and glycoproteins, nucleic acids, e.g., DNA, RNA and PNA (peptide nucleic acid). Examples of small molecules are peptides, peptidomimetics (e.g., peptoids), amino acids, amino acid analogs, polynucleotides, polynucleotide analogs, nucleotides, nucleotide analogs, organic or inorganic compounds e.g., heteroorganic or organometallic compounds. A test compound can be the only substance assayed by the method described herein. Alternatively, a collection of test compounds can be assayed either consecutively or concurrently by the methods described herein. Exemplary test compounds can be obtained from a combinatorial chemical library including peptide libraries (*see, e.g.,* U.S. Patent 5,010,175, Furka, *Int. J. Pept. Prot. Res.* 37:487-493 (1991) and Houghton *et al., Nature* 354:84-88 (1991)), peptoids (e.g., PCT Publication No. WO 91/19735), encoded peptides (e.g., PCT Publication No. WO 93/20242), random bio-oligomers (e.g., PCT Publication No. WO 92/00091), benzodiazepines (e.g., U.S. Pat. No. 5,288,514), diversomers such as hydantoins, benzodiazepines and dipeptides (Hobbs *et al., Proc. Nat. Acad. Sci. USA* 90:6909-6913 (1993)), vinylogous polypeptides (Hagihara *et al., J. Amer. Chem. Soc.* 114:6568 (1992)), nonpeptidal peptidomimetics with glucose scaffolding (Hirschmann *et al., J. Amer. Chem. Soc.* 114:9217-9218 (1992)), analogous organic syntheses of small compound libraries (Chen *et al., J. Amer. Chem. Soc.* 116:2661 (1994)), oligocarbamates (Cho *et al., Science* 261:1303 (1993)), and/or peptidyl phosphonates (Campbell *et al., J. Org. Chem.* 59:658 (1994)), nucleic acid libraries (*see* Ausubel, Berger and Sambrook, all *supra*), peptide nucleic acid libraries (*see, e.g.,* U.S. Patent 5,539,083), antibody libraries (*see, e.g.,* Vaughn *et al., Nature Biotechnology,* 14(3):309-314 (1996) and PCT/US96/10287), carbohydrate libraries (*see, e.g.,* Liang *et al., Science,* 274:1520-1522 (1996) and U.S. Patent 5,593,853), small organic molecule libraries (*see, e.g*., benzodiazepines, Baum C&EN, Jan 18, page 33 (1993); isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337; benzodiazepines, 5,288,514, and the like).

The test compound or compounds can be screened individually or in parallel. A compound can be screened by being monitored the level of expression of one or more nucleic acids selected from Group I, II, III, or IV. Comparing a compound-associated expression profile to a reference profile can identify the ability of the compound to modulate metastastic gene expression. The expression profile can be a profile of at least two cell lines which are clonally related. Examples of the cell lines are human lung adenocarcinoma cell lines of different invasive and metastatic capacities, e.g., CL₁₋₀ and its sublines (e.g., CL₁₋₁ and CL₁₋₅). An example of the parallel screening is a high throughput drug screen. A high-throughput method can be used to screen large libraries of chemicals. Such libraries of test compounds can be generated or purchased e.g., from Chembridge Corp., San Diego, CA. Libraries can be designed to cover a diverse range of compounds. For example, a library can include 10,000, 50,000, or 100,000 or more unique compounds. Alternatively, prior experimentation and anecdotal evidence, can suggest a class or category of compounds of enhanced potential. A library can be designed and synthesized to cover such a class of chemicals. A library can be tested on cell lines, such as CL₁₋₀ and its sublines, and gene expression levels can be monitored. Regardless of a method used for screening, compounds that alter the expression level are considered "candidate" compounds or drugs. Candidate compounds are retested on metastastic cells, or tested on animals. Candidate compounds that are positive in a retest are considered "lead" compounds.

Once a lead compound has been identified, standard principles of medicinal chemistry can be used to produce derivatives of the compound. Derivatives can be screened for improved pharmacological properties, for example, efficacy, pharmacokinetics, stability, solubility, and clearance. The moieties responsible for a compound's activity in the assays described above can be delineated by examination of structure-activity relationships (SAR) as is commonly practiced in the art. A person of ordinary skill in pharmaceutical chemistry could modify moieties on a lead compound and measure the effects of the modification on the efficacy of the compound to thereby produce derivatives with increased potency. For an example, see Nagarajan *et al.* (1988) *J*. *Antibiot.* 41: 1430-8. Furthermore, if the biochemical target of the lead compound is known or determined, the structure of the target and the lead compound can inform the design and optimization of derivatives. Molecular modeling software is commercially available (e.g., Molecular Simulations, Inc.).

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications, including patents, cited herein are hereby incorporated by reference in their entirety.

### EXAMPLE

### Materials

**Cell Lines.** Human lung adenocarcinoma cell lines of different invasive and metastatic capacities (CL₁₋₀ and its sublines, CL₁₋₁ and CL₁₋₅) were grown in RPMI medium with 10% fetal bovine serum (FBS) at 37 °C, 20% O₂, and 5% CO₂. See Chu *et al*. (1997) *Am. J. Respir. Cell Mol. Biol*. 17: 353-360.

***In Vitro* Invasion Assay.** CL₁₋₅ cells were injected into the tail veins of SCID mice to obtain a more invasive cell line than the just described CL₁ series. A highly metastatic cell line was isolated and cloned from the cancer lesion formed in the lung of mice. After four-repeated *in vivo* selection, the cell line was designated as CL₁₋₅₋F₄ and incorporated into the panel of cell lines for microarray analysis.

Invasiveness of the CL₁ series of cell lines was examined by using membrane invasion culture system (MICS). In the MICS system, a polycarbonate membrane containing 10 µm pores (Nucleopore Corp., Pleasanton, CA) was coated with a mixture of laminin (50 µg/ml; Sigma Chemical Co., St. Louis, MO), type IV collagen (50 µg/ml; Sigma), and gelatin (2 mg/ml; Bio-Rad, Hercules, CA) in 10 mM glacial acetic acid solution. The membrane was placed between upper- and lower- well plates of a MICS chamber. CL₁ cell line series were then re-suspended in RPMI containing 10% NuSerum and seeded into the upper wells of the chamber (5×10⁴ cells/well). After incubating for 24 hours at 37°C, cells that invaded through the coated membrane were removed from the lower wells with 1 mM ethylene diamine tetraacetic acid (EDTA) in phosphate-buffered saline (PBS) and dot-blotted onto a polycarbonate membrane with 3 µm pores. After fixation in methanol, blotted cells were stained with Liu stain (Handsel Technologies, Inc., Taipei, Taiwan) and the cell number in each blot was counted under a microscope. Each experiment was repeated for three times.

**Tracheal Graft Invasion Assay.** A tracheal graft invasion assay was carried out to confirm the *in vitro* selected lung cancer cell lines with different invasive/metastatic potentials also possess invasive ability *in vivo*. Rat tracheas were isolated from Sprague-Dawley (SD) rats weighing around 200 gm. The airway epithelial cells of the tracheas were denuded by repetitive freeze-and-thaw procedures for three times at -70°C. The CL₁₋₀, CL₁₋₁ and CL₁₋₅ cells were cultured to sub-confluence before they were harvested. 10⁶ cells from each cell line were then injected into the isolated rat tracheas. The upper and lower ends of the tracheas were tightened with threads and implanted subcutaneously in SCID mice. Each cell line was sealed in three different trachea grafts and each SCID mouse was implanted with one graft. The SCID mice were sacrificed four weeks later and the tracheal grafts were taken out for histological examination. The tumor part of the tracheal graft was sliced at 1 mm intervals. At least three sections were examined for the presence of basement membrane invasion. All animal experiments were performed in accordance with the animal guidelines at The Department of Animal Care, Institute of Biomedical Sciences, Academia Sinica, Taipei, Taiwan.

**Biotinylated Probe Preparation and Microarray Hybridization**. Five micrograms of the mRNAs derived from each lung cancer cell line were labeled with biotin during reverse transcription. See Chen *et al*. (1998) *Genomics* 51: 313-324; and Hong *et al*. (2000) *Am. J. Respir. Cell Mol. Biol.* 23: 355-363. The microarrays (18 mm by 27 mm) carrying 9,600 PCR-amplified cDNA fragments were prepared on Nylon membranes by an arraying machine built in-house. The 9600 non-redundant expressed sequence tag (EST) clones were IMAGE human cDNA clones each representing a putative gene cluster with an assigned gene name in the Unigene clustering (Schuler (1997) *J*. *Mol. Med.* 75: 694-698). All experiments of hybridization were performed in triplicate individually. The details of probe preparation, hybridization, and color development were also described previously.

The microarray images were digitized by using a drum scanner (ScanView, Foster City, CA). Image analysis and spot quantification were done by the MuCDA program written in-house. The program is available via anonymous ftp at ftp://genestamp.ibms.sinica.edu.tw/marray/software/. The microarray images can also be processed by commercial image processing programs and other available microarray image analysis programs.

**Northern hybridization.** To confirm the results of gene detection by the cDNA microarray, sixteen of differentially expressed cDNA clones including ten clones of ascending trend and six clones of descending trend in metastasis were selected from cluster analysis of array data, and the entire inserts of the clones were individually PCR-amplified to serve as probes for Northern hybridization. The hybridization and washing procedures were carried out by standard protocol and described in our previous report (Hong *et al.* (2000) *Am. J. Respir. Cell Mol. Biol.* 23: 355-363).

**Flow cytometric assay.** The adenocarcinoma cell sublines, CL₁₋₀, CL₁₋₁, CL₁₋₅ and CL₁₋₅-F₄, were subjected to indirect immunofluorescence staining for the expression of surface tumor-associated antigen L6, integrin α-3 and integrin α-6 using murine mAb against human tumor-associated antigen L6 (ATCC, Manassas, VA), integrin α-3 (Chemicon, Temecula, CA) and integrin α-6 (BQ16; Acell Co., Bayport, MN) respectively. The fluorescence intensity was analyzed by FACStar (Becton-Dickinson, Mountain view, CA).

**Statistical Analysis.** A cluster analysis method to identify invasion-associated genes was performed on the microarrays. Gene expression data obtained from the microarray experiments were processed and normalized using the protocol and program described by Iyer, V.R. *et al*. (1999) *Science* 283: 83-87. Genes were clustered into groups on the basis of expression profiles by self-organizing maps (SOMs) algorithm as described by Tamayo P. *et al*. (1999) *Proc. Natl. Acad. Sci. USA* 96: 2907-2912. After cluster analysis by the SOM method, genes whose expression profiles correlate either positively or negatively with the invasiveness of cell lines were identified.

A repeated measurement analysis of variance (ANOVA test) was performed to determine any significant difference between the numbers of invasion foci formed in tracheal grafts. Data from three experiments in duplicates was analyzed by ANOVA test (Excel, Microsoft, Taiwan) to determine any significant difference.

### Results

Invasiveness abilities were measured in the four human lung adenocarcinoma cell lines, CL₁₋₀, CL₁₋₁, CL₁₋₅ and CL₁₋₅₋F₄. Cells invading through the coated membrane were harvested and counted. The cell counts were: CL₁₋₀: 202 ± 16; CL₁₋₁: 1491 ± 202; CL₁₋₅: 3865 ± 530; and CL₁₋₅-F₄: 4115 ± 507. The invasiveness of the four cell lines were as expected and followed a trend of: CL₁₋₅₋F₄ ≥ CL₁₋₅ > CL₁₋₁ > CL₁₋₀.

The invasiveness of the four adenocarcinoma cell lines was confirmed to have equivalent *in vivo* invasiveness by the tracheal graft invasion assay. After four weeks, the human airway epithelial cells were repopulated on the rat tracheal basement membrane. The repopulated airway epithelial cells revealed pseudostratified columnar epithelium with mucus and ciliary differentiation. After tracheal graft injected with CL₁₋ ₀ cells, tumor formation was evident. However, histochemical staining of the control rat trachea without tumor cell injection and without epithelial cells on the basement membrane revealed no invasion of the basement membrane. When tracheal graft was injected with CL₁₋₅ cells, invasion of the basement membrane was clearly evident, in addition to tumor formation in rat trachea. The invasion foci in three sections of the three cell lines were also calculated. The total invasion foci per graft for CL₁₋₀, CL₁₋₁ and CL₁₋₅ cells were 0.0, 0.7 ± 0.5 and 4.0 ± 2.0, respectively (ANOVA test: α=0.05, *P*=0.0133).

Biotin-labeled probes deriving from mRNAs of cell lines of varying invasiveness were hybridized to microarrays with 9,600 putative genes to profile the gene expression patterns. Microarray images showed the gene expression patterns for a series of lung adenocarcinoma cell lines. The trend of gene expression level changes could clearly be seen. It has been observed that the expression levels of the calcyclin gene correlated positively with cell line invasiveness, and the expression levels of the AXL gene also correlated positively with invasiveness.

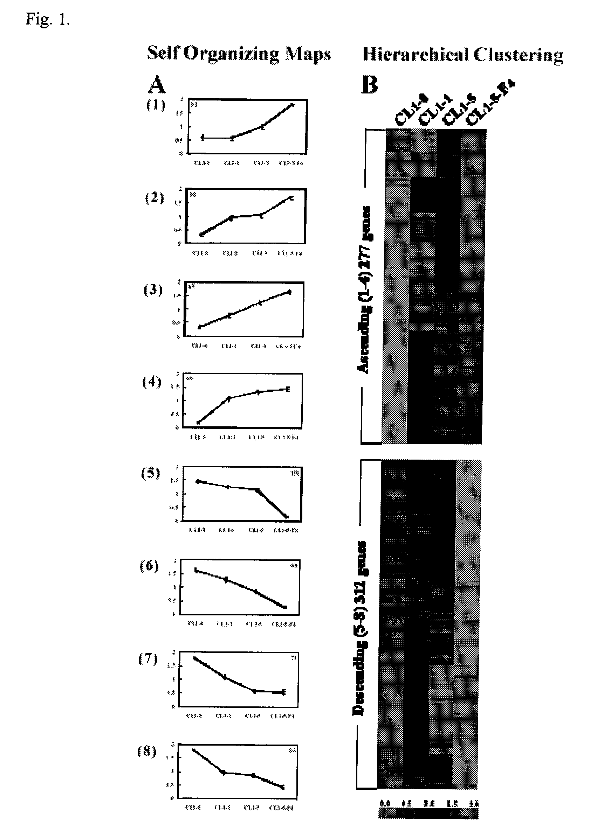

In order to identify all possible metastasis-associated genes from the 9,600-feature nucroarray, a cluster analysis on the expression profiles of the four lung adenocarcinoma cell lines was performed. Of the 9,600 putative genes, 8,525 had statistically significant expression values and their expression profiles were grouped into 100 clusters. To avoid confusion of negative values in expression patterns, the scale value of normalization, from -1 to +1, was shift to a positive value, from 0 to +2. Four exemplary clusters (No.1-No.4, shown in FIG 1) correlated positively with the invasiveness of the cell lines. The four clusters contained expression profiles of 61,50,67, and 99 genes, respectively. Another four exemplary clusters (No.5-No.8, shown in FIG. 1) correlated negatively with invasiveness and each cluster contained 110, 68, 71, and 63 genes, respectively. The gene expression profiles (277 positively correlated genes and 312 negatively correlated genes) were rearranged by hierarchical cluster analysis using the average linkage method.

To substantiate the results of the microarray studies, a Northern-blotting analysis was performed. Ten genes having ascending expression containing five sequence-verified known genes (i.e., calcyclin, AXL, tumor-associated antigen L6, Metallothionein I-B, and RTP) and five anonymous genes (i.e., EST-T40480, EST-T70568, EST-R16261, EST-N20320, and EST-T52774) whose expression had a positive correlation were selected. These ten genes had higher expression levels in the more invasive cell line (CL₁₋₅-F₄). Another six genes having descending expression, five of which are sequence-verified known genes (i.e., proteoglycan I secretory granule, TFIID I, DnaJ-like heat shock protein 40, phosphoenolpyruvate carboxykinase 2, and soluble VEGF receptor) and one is anonymous gene (EST-H04819) whose expression had a negative correlation with the invasiveness of adenocarcinoma cell lines, were also selected to perform Northern blotting. These six genes were highly expressed in the less invasive cell line (CL₁₋₀). The results of Northern blotting analysis were consistent with those from the microarray studies. Radio-labeled GAPDH and Gβ-like protein were used as internal controls.

To demonstrate the protein expression of identified genes was also consistent with microarray analysis, three antibodies, tumor-associated antigen L6, integrin α-3 and integrin α-6, were used to carry out flow cytometric analysis across all four CL₁ sublines respectively. Each experiment was carried out in triplicate. The average background of fluorescence was 3.3 ± 0.64 (arbitrary fluorescence intensity). The antibody against tumor-associated antigen L6 was used to quantify protein expression level, it was obvious that the peak was shifted from CL₁₋₀ (18 ± 10.0) to CL₁₋₅-F₄ (233 ± 36.9) and the differentially expressed ratio of CL₁₋₅-F₄ to CL₁₋₀ was 12.94. The antibody against integrin α-3 made the peak shift from CL₁₋₀ (3 ± 0.6) to CL₁₋₅-F₄ (49 ± 17.3) and the differentially expressed ratio was 16.33. The antibody against integrin α-6 made the peak shift from CL₁₋₀ (14 ± 2.8) to CL₁₋₅-F₄ (53 ± 21.7) and the differentially expressed ratio was 3.79. These results demonstrated that flow cytometric analysis of protein were consistent with microarray analysis or Northern blotting analysis

### OTHER EMBODIMENTS

All of the features disclosed in this specification may be used in any combination. Each feature disclosed in this specification may be replace by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Accordingly, other embodiments are also within the scope of the following claims.

## Claims

1. A method for evaluating a sample, the method comprising:
determining the abundance of at least one nucleic acid selected from Group I and/or II in a first sample; and
determining the abundance of the at least one nucleic acid in a second sample that comprises normal cells or tumor cells;
comparing the abundance of the at least one nucleic acid in the first sample to the abundance of the at least one nucleic acid in the second sample, and
categorizing the sample as having tumor invasive or metastatic potential based on results of the comparing.

2. A method for evaluating a sample, the method comprising:
determining the abundance of at least one nucleic acid selected from Group I and/or II in a first sample; and
determining the abundance of the at least one nucleic acid in a second sample that comprises normal cells;
determining the abundance of the at least one nucleic acid in a third sample that comprises tumor cells;
comparing the abundance of the at least one nucleic acid in the first sample to the abundance of the at least one nucleic acid in the second sample and abundance of the at least one nucleic acid in the second sample the third sample, and
categorizing the first sample as having tumor invasive or metastatic potential based on results of the comparing.

3. The method of claim 2 wherein increased similarity between the first sample and third sample relative to similarity between the first sample and the second sample categorizes the first sample as having tumor invasive or metastatic potential.

4. The method of claim 1 or 2, wherein the at least one nucleic acid comprises at least ten nucleic acids selected from Groups I and/or II.

5. The method of claim 4, wherein the at least one nucleic acid comprises at least ten nucleic acids selected from Group I.

6. The method of claim 1 or 2, wherein the at least one nucleic acid comprises one or more nucleic acids selected from the group consisting of: EST-T70568, EST-N20320, EST-T52774, proteoglycan I secretory granule (W19210), DnaJ-like heat shock protein 40 (A084517), and phosphoenolpyruvate carboxykinase 2 (R40253).

7. A method of evaluating a sample, the method comprising:
identifying a expression profile that represents the levels of protein or mRNA expression from at least two genes selected from Group I and/or II in a sample; and
comparing the sample expression profile to at least one reference expression profile;
wherein each of the sample expression profile and the reference expression profile includes a plurality of values, each of the values is an assessment of the abundance of (1) an mRNA transcribed from a gene selected from Group I and/or II; or (2) a polypeptide encoded by the gene.

8. The method of claim 7, wherein each of the sample expression profile and the reference expression profile includes a plurality of values for 50% of the members of Group I.

9. The method of claim 8, wherein each of the sample expression profile and the reference expression profile includes a plurality of values for 80% of the members of Group I.

10. The method of claim 7, wherein each of the sample expression profile and the reference expression profile includes a plurality of values for 20% of the members of Group II.

11. The method of claim 9, wherein the comparing comprises evaluating a Euclidean distance.

12. The method of claim 9, wherein the comparing comprises evaluating a correlation coefficient.

13. The method of claim 9, wherein the reference profile is a profile of a non-tumorous cell.

14. The method of claim 9, wherein the reference profile is a profile of a tumor cell.

15. The method of claim 14, wherein the tumor cell is a cultured lung adenocarcinoma cell.02

16. A method for diagnosing tumor invasive potential or metastatic development in a subject, the method comprising:
providing a sample from the subject;
determining a protein or mRNA expression profile of the sample;
comparing the expression profile to a reference profile for a non-metastatic cell; and
categorizing the subject as having tumor invasive potential or metastatic development when the sample expression profile is found to be altered relative to the reference expression profile, wherein each of the sample expression profile and the reference expression profile includes one or more values representing the levels of expression of one or more nucleic acids selected from Group I and/or II.

17. The method of claim 16, wherein each of the sample expression profile and the reference expression profile includes one or more values representing the levels of expression of 50% of nucleic acids selected from Group I.

18. The method of claim 16, wherein the sample comprises a biopsy.

19. The method of claim 16, wherein the sample comprises lung tissue or lung cells.

20. The method of claim 19, wherein the expression profile is an mRNA expression profile and the expression profile is determined using a nucleic acid array.

21. A method for screening for a test compound useful in the prevention or treatment of tumor metastasis, comprising:
providing a reference expression profile;
contacting the test compound to a cell;
determining a compound-associated expression profile for the contacted cell; and comparing the compound-associated expression profile to the reference profile;
wherein each of the compound-associate expression profile and the reference expression profile includes one or more values representing the level of expression of one of more nucleic acids selected from Group I and/or Group II.

22. The method of claim 21, wherein each of the compound-associated profile and the reference expression profile includes one or more values representing the levels of expression of at least 50% of nucleic acids selected from Group I.

23. The method of claim 22, wherein each of the compound-associated profile and the reference expression profile includes one or more values representing the levels of expression of at least 80% of nucleic acids selected from Group I.

24. The method of claim 21, wherein each of the compound-associated profile and the reference expression profile includes one or more values representing the levels of expression of 20% of nucleic acids selected from Group II.
